# EUROPEAN PATENT APPLICATION

(11) **EP 1 704 866 A2**
(43) Date of publication of application: **27.09.2006**
(21) Application number: 06115688.1
(22) Date of filing: 07.03.2001
(51) Int. Cl.: A61K 38/18, A61L 27/54, A61P 19/04

(54) **TGFbeta superfamily proteins for biological anchoring of connective tissue to bone**

(30) Priority: 09.03.2000 US 523923
(62) Divisional of application: 01918377.1
(71) Applicant: Zimmer Orthobiologics, Inc., Austin, Texas 78726-4050 (US)
(72) Inventor: Atkinson, Brent, Lakewood, CO 80215 (US); Benedict, James, J., Arvada, CO 80007 (US)
(74) Representative: Harrison Goddard Foote

(57) **Abstract**

There is disclosed the use of a mixture of proteins for the manufacture of a medicament for enhancing an attachment of bone to connective tissue, the mixture comprising at least four different members of the TGFβ superfamily proteins.

## Description

### FIELD OF THE INVENTION

The present invention relates to a product for enhancing an attachment of connective tissue to bone, whereby the product induces the formation of a bone-cartilage-connective tissue interface at a site of attachment of connective tissue to bone. The present invention also relates to methods of using such a product to repair connective tissue-to-bone attachments.

### BACKGROUND OF THE INVENTION

Current methods for the fixation of tendon or ligament to bone utilize mechanical fixation. However, none of these methods control or enhance the biological healing response and none of these methods optimize the biological component of fixation. Moreover, no current method of reconstruction exists that recreates the normal morphology of tendon or ligament to bone. Various operative techniques do not recreate the complex fan morphology or the transition zones of fibrocartilage and calcified cartilage between the tendon or ligament and the bone. As leisure time, affluence, and an emphasis on fitness and high impact sports such as football and skiing have increased, a significant rise in injuries involving tendon and ligament attachments to bone has been observed. Tissues that are frequently damaged and/or severed by such injuries include the anterior cruciate ligament (ACL) and the tendons of the rotator cuff.

The anterior cruciate ligament (ACL) is located in the center of the knee joint between the tibia and the condyles of the femur. Functionally, the ACL prevents backward dislocation of the femur, forward dislocation of the tibia, and excessive extensor or rotational movements. A significant rise in ACL injuries has been observed over the last decades as activities that place strain on this ligament have increased. Currently utilized surgical procedures for ACL reconstructions are described by Jackson (DW Jackson and PR Kurzweil, Chapter 8 in Master Techniques in Orthopaedic Surgery, Reconstructive knee surgery, Raven Press, NY, 1995). Briefly, femoral and tibial tunnels are created, and the graft is harvested and fixed to both the femoral and tibial tunnels. In the majority of reconstructions, an autologous graft is harvested from the hamstring, quadriceps or patellar tendon, although allograft material is also used in a small percentage of cases. Synthetic (e.g., non-human derived) material has also been used to substitute for the ACL (Bolton et al., Clin. Orthop., 196:202-213, 1985). In addition, reconstructions have used grafts derived from xenograft material and/or subintestinal submucosa and/or collagen-derived grafts. Currently utilized fixation methods include interference screw, endobutton, suture, compression screw, anchor, and other methods which may consist of resorbable or non-resorbable materials.

The tendons of the rotator cuff, the supraspinatus, infraspinatus, subscapularis, and teres minor, support the joint capsule and limit the range of rotator cuff movement. These tendons insert around the head of the humerus, with the supraspinatus, infraspinatus, and teres minor tendons attaching to the greater tuberosity and the subscapularis tendon to the lesser tuberosity. Fibrocartilage is often found at the tendon to bone insertion site. The function of the fibrocartilage has been hypothesized to be similar to the function of the grommet that is placed around an electrical cord to prevent shearing damage to the wires where it enters the plug (Benjamin et al., "Functional and Developmental Anatomy of Tendons and Ligaments." In Gordon SL, Blair SJ, Fine LJ (eds): Repetitive Motion Disorders of the Upper Extremity; Rosemont, IL, American Academy of Orthopaedic Surgery, 1995). The current methods for rotator cuff repair are thoroughly described by Fu (Operative Techniques in Orthopaedics, "Treatment of Rotator Cuff Injuries"; Editor F.H. Fu, guest editor GR Williams, vol. 8, no. 4, 1998). Generally, a bone trough into the cancellous bone is created just medial to the greater tuberosity, bone tunnels are drilled along the trough, and transosseous sutures were utilized to secure the tendon to the bone. In addition, the stitch pattern can be modified. Suture anchors/harpoons can be used as an alternative to transosseous sutures (Operative Techniques in Orthopaedics, 1998, *ibid.*).

In the 1960's, demineralized bone matrix was observed to induce the formation of new cartilage and bone when implanted in ectopic sites (Urist, Science, 150:893-899, 1965). The components responsible for the osteoinductive activities were termed Bone Morphogenetic Proteins (BMP). At least seven individual BMPs were subsequently identified from bone and were designated BMP 1-7. Amino acid analysis revealed that six of the seven BMPs were related to each other and to other members of the TGF-β superfamily (Celeste et al., Proc. Natl. Acad. Sci. USA, 87:9843-9847,1990; Wozney et al., Science, 242:1528-1534, 1988). During endochondral bone formation, TGF-β family members direct a cascade of events that includes chemotaxis, differentiation of pluripotent cells to the cartilage lineage, maturation of chondrocytes to the hypertrophic stage, mineralization of cartilage, replacement of cartilage with bone cells, and the formation of a calcified matrix (Reddi, Cytokine & Growth Factor Reviews, 8:11-20, 1997). Although individual, recombinant BMPs can induce these events, the presence of multiple TGF-β family members in bone underscores the complexity involved in natural osteogenesis.

More recently, additional members of the BMP family have been discovered, some of which have been designated to the growth and differentiation factor (GDF) family. To clarify the nomenclature, BMP-12, BMP-13, and BMP-14 are also known as GDF-7, -6, and -5, respectively (AH Reddi, Nat. Biotech., 16:247-252, 1998). Also, GDF-5, -6, and -7 are known as CDMP-1, -2, and -3, respectively. In addition, MP-52 is the same molecule as GDF-5.

Bone Protein (Sulzer Orthopedics-Biologics, Denver, CO), BP, is a naturally derived mixture of proteins isolated from demineralized bovine bones that has osteogenic activity *in vitro* and *in vivo.* In the rodent ectopic model, BP induces endochondral bone formation or bone formation through a cartilage intermediate (Damien et al., J. Biomed. Mater. Res., 24:639-654, 1990). *In vitro,* BP promotes stem cell differentiation to cartilage (Atkinson et al., J. Cell. Biochem., 65:325-339, 1997). The process of purifying BP and the method of forming bone in the body in combination with calcium carbonate is described in U.S. Patent Nos. 5,290,763, 5,371,191, and 5,563,124.

A rat subcutaneous model can be utilized to demonstrate similarities and differences among recombinant BMP molecules and/or bone-derived compositions. In the Rosen modified Sampath-Reddi rodent assay (Operative Techniques in Orthopaedics, 1998, *supra*), BMP-12, BMP-13 and MP-52 induced no bone or cartilage after 10 days. Instead, these factors produced tissue resembling embryonic tendon (Wolfman et al., Clin. Invest., 100(2):321-30, 1997). Celeste et al. describe a method for using BMP-12, BMP-13, and/or MP-52 (i.e., BMP-14) to induce the formation of tendon and/or ligament tissue (U.S. Patent No. 5,658,882 and WO95/16035). Using a modified rat ectopic implant assay, Celeste et al. show that recombinant BMP-12 induces the formation of tissue resembling embryonic tendon and no cartilage or bone formation. In contrast, BMP-2 containing implants in this rodent ectopic assay showed cartilage and bone formation, but did not contain tendon/ligament-like tissue (Celeste et al., U.S. Patent No. 5,658,882).

The bone tunnel model is a convenient model for studying tendon or ligament attachment to bone (Rodeo et al., 1993, J. Bone Joint Surgery 75-A(12):1795-1803). In this model, the long digital extensor tendon is detached from the femoral condyle, a tunnel is created in the tibia, the tendon is pulled through a tunnel in the proximal part of the tibia, and it is fixed to the periosteum. In this model, Wozney et al., describe the use of purified bone morphogenetic protein for the regeneration of the functional attachment of connective tissue to bone (WO96/39169). The natural progress of tendon to bone healing was observed histologically and mechanically over time. However, although histological analysis demonstrated that BMP-2 induced a bone-tendon interface, the preferred fibrocartilaginous zone was not present. In a publication of experiments using BMP-2 to enhance tendon healing in a bone tunnel, Rodeo et al. reported that higher doses of BMP-2 induced resorption of the lamellar bone surrounding the bone tunnel, which is undesirable, with concomitant formation of fine, new bone trabeculae (Rodeo et al.,1999, Amer. J. Orthopaedic Society for Sports Medicine 27:476-488). In addition, this report indicated, as in WO 96/39169, that although a bone-tendon interface was formed, cartilage formation in the interface was not observed.

Additional studies have demonstrated the effect of growth factors on tendon to tendon or ligament to ligament healing. Woo et al. utilized an *in vitro* ligament model to show that epidermal growth factor-β1 (TGF-β1) promotes extracellular matrix synthesis (Woo et al., Med. Biol. Eng. Comput., 36(3):359-64,1998*).* Using a rabbit ruptured MCL model, tensile testing revealed that platelet-derived growth factor BB (PDGF-BB) significantly increased ultimate load, ultimate elongation and energy absorbed to failure values compared to controls (Woo et al., 1998, *ibid.*). Furthermore, Aspenberg and Forslund demonstrated that GDF 5 and 6 (i.e., BMP-13 and BMP-14) increased the tensile strength of the regenerated tendon in a transected the Achilles tendon rats model. (Woo et al., 1998, *ibid.*). In addition, exogenously added bone morphogenetic protein caused ossification of the ligamentum flavum in mice (Woo et al.,1998, *ibid*). However, as discussed above, these studies have not been able to demonstrate a reconstruction/repair of connective tissue-cartilage-bone interface that replicates the natural ontogeny of such an interface to a degree suitable for use in clinical *in vivo* tendon/ligament repair.

In summary, despite considerable research directed to the repair of injuries to the attachment of tendon and/or ligament to bone, there remains a need in the art for a product and method which not only produces a functional attachment of tendon or ligament to bone, but which also induces the formation of an attachment of tendon/ligament to bone which more faithfully replicates the natural (i.e., endogenous) ontogeny of the attachment. Replication of the natural ontogeny of tendon and/or ligament to bone would provide a result which includes induction of a greater quantity of bone which appears faster and is in closer proximity to the connective tissue, an increase in the fixation strength of the attachment at earlier time points, and an increase in the fixation strength in patients with degenerative tendon and/or bone pathology.

### SUMMARY OF THE INVENTION

The present invention generally relates to a product and method for enhancing the attachment of connective tissue to bone. The connective tissue is selected from the group of tendon, ligament and cementum and in a preferred embodiment, is selected from the group of tendon and ligament.

The product of the present invention comprises: (a) a matrix configured to interface between connective tissue and bone; and, (b) a composition comprising a mixture ofproteins associated with the matrix effective to induce the formation of a bone-cartilage-connective tissue interface at a site of attachment of connective tissue to bone. The mixture of proteins includes, but is not limited to: transforming growth factor β1 (TGFβ1), bone morphogenetic protein (BMP)-2, BMP-3, and BMP-7. The quantity of the TGFβ1 in the mixture is from about 0.01 % to about 10% of total proteins in the mixture; the quantity of the BMP-2 in the mixture is from about 0.01 % to about 10% of total proteins in the mixture; the quantity of the BMP-3 in the mixture is from about 0.1% to about 15% of total proteins in the mixture; and, the quantity of the BMP-7 in the mixture is from about 0.01% to about 10% of total proteins in the mixture. In a one embodiment, the quantity of the TGFβ1 in the mixture is from about 0.05% to about 1% of total proteins in the mixture. In one embodiment, the quantity of the BMP-2 in the mixture is from about 0.1% to about 5% of total proteins in the mixture. In another embodiment, the quantity of BMP-3 in the mixture is from about 0.1 % to about 10% of total proteins in the mixture. In yet another embodiment, the quantity of the BMP-7 in the mixture is from about 0.1% to about 5% of total proteins in the mixture.

In one embodiment, the mixture of proteins further comprises a protein selected from the group of TGFβ2, TGFβ3, BMP-4, BMP-5, BMP-6, BMP-8, BMP-9, and cartilage-derived morphogenetic protein (CDMP). In one aspect of this embodiment, the TGFβ2 comprises from about 0.5% to about 12% of the mixture of proteins; the TGFβ3 comprises from about 0.01 % to about 15% of the mixture of proteins; the BMP-4 comprises from about 0.01% to about 1% of the mixture of proteins; the BMP-5 comprises from about 0.01% to about 1% of the mixture of proteins; the BMP-6 comprises from about 0.01% to about 1% of the mixture of proteins; and/or the CDMP comprises from about 0.01 % to about 1% of the mixture of proteins. In one embodiment, the mixture of proteins further comprises a CDMP protein in a quantity of from about 0.01% to about 10% of total proteins in the mixture.

In one embodiment, the mixture ofproteins further comprises at least one bone matrix protein selected from the group consisting of osteocalcin, osteonectin, bone sialoprotein (BSP), lysyloxidase, cathepsin L pre, osteopontin, matrix GLA protein (MGP), biglycan, decorin, proteoglycan-chondroitin sulfate III (PG-CS III), bone acidic glycoprotein (BAG-75), thrombospondin (TSP) and fibronectin. The bone matrix protein typically comprises from about 20% to about 98% of the mixture of proteins; in one embodiment, the bone matrix protein comprises from about 40% to about 98% of the mixture of proteins. In a preferred embodiment, the bone matrix proteins comprise: osteocalcin, osteonectin, bone sialoprotein (BSP), lysyloxidase, and cathepsin L pre.

In one embodiment, the mixture of proteins further comprises at least one growth factor protein selected from the group consisting of fibroblast growth factor-I (FGF-I), FGF-II, FGF-9, leukocyte inhibitory factor (LIF), insulin, insulin growth factor I (IGF-I), IGF-II, platelet-derived growth factor AA (PDGF-AA), PDGF-BB, PDGF-AB, stromal derived factor-2 (SDF-2), pituitary thyroid hormone (PTH), growth hormone, hepatocyte growth factor (HGF), epithelial growth factor (EGF), transforming growth factor-α (TGFα) and hedgehogproteins. The growth factor protein typically comprises from about 0.01% to about 50% of the mixture of proteins, and in one embodiment, the growth factor protein comprises from about 0.05% to about 25% of the mixture of proteins, and in another embodiment, the growth factor protein comprises from about 0.1% to about 10% of the mixture of proteins. Preferably, the growth factor protein is fibroblast growth factor-I (FGF-I). In this embodiment, the FGF-I comprises from about 0.001% to about 10% of the mixture of proteins.

In another embodiment, the composition further comprises one or more serum proteins. Preferably, the serum proteins are selected from the group consisting of albumin, transferrin, α2-Hs GlycoP, IgG, α1-antitrypsin, β2-microglobulin, Apo A1 lipoprotein (LP) and Factor XIIIb. Even more preferably, the serum proteins are selected from the group consisting of albumin, transferrin, Apo A1 LP and Factor XIIIb.

In one embodiment of the present invention, the mixture of proteins comprises TGFβ1, TGFβ2, TGFβ3, BMP-2, BMP-3, BMP-4, BMP-5, BMP-6, BMP-7, CDMP, FGF-I, osteocalcin, osteonectin, BSP, lysyloxidase, cathepsin L pre, albumin, transferrin, Apo A1 LP and Factor XIIIb. In another embodiment, the mixture of proteins comprises Bone Protein (BP). Preferably, the composition has an identifying characteristic selected from the group consisting of an ability to induce cellular infiltration at the site of attachment, an ability to induce cellular proliferation at the site of attachment, an ability to induce formation of bone, an ability to induce formation oftendon, and an ability to induce formation of cartilage. In a preferred embodiment, the product does not substantially induce bone resorption at the site of attachment. In another embodiment, the composition, in a rat subcutaneous assay, induces an ordered ring of bone formation peripherally around an implanted collagen sponge and induces an ordered ring of collagen formation interior to the ordered ring of bone formation. In yet another embodiment, the mixture of attachment proteins, when used at a concentration of about 10 µg per 6.5-7.3 mg of bovine tendon collagen in a rat subcutaneous assay, induces a bone score of at least about 2.0, using a bone grading scale set forth in Table 1, and induces a cartilage score of at least about 1.5, using a cartilage grading scale set forth in Table 8.

In the product of the present invention, the composition is typically at a concentration of from about 0.5% to about 33% by weight of the product, and in one embodiment, is at a concentration of from about 1% to about 20% by weight of the product.

The matrix component of the product of the present invention is, in one embodiment, bioresorbable. Preferably, the matrix is porous. The matrix preferably comprises a material selected from the group consisting of a synthetic polymeric material and a ground substance. In one aspect, the matrix comprises a material selected from the group consisting of a sponge, a membrane, a film and a gel. In a preferred embodiment, the matrix comprises collagen from bovine tendon. In one embodiment, the matrix conforms to a site of attachment of tendon or ligament to a bone concavity.

Another embodiment of the present invention relates to a method for enhancing an attachment of bone to connective tissue selected from the group of tendon, ligament and cementum. Such a method includes the steps of implanting and fixing at a site of attachment of bone to connective tissue, a product of the present invention as described above. The method of the present invention is particularly useful when the site of attachment is a site of attachment of an anterior cruciate ligament to bone, when the site of attachment is a site of attachment of a tendon selected from the group consisting of supraspinatus, infraspinatus, subscapularis and teres minor, to bone; when the site of attachment is a site of attachment of an ulnar collateral ligament to bone; when the site of attachment is a site of attachment of a lateral ankle ligament to bone; and/or when the site of attachment is a site of attachment of cementum to alveolar bone.

Preferably, the product is fixed into the lesion by an attachment means selected from the group of interference screw, endobutton, bioresorbable sutures, compression screw, non-resorbable sutures, press-fitting, arrows, nails, and T-fix suture anchor devices.

In one embodiment, the attachment matrix is configured as a sheet. In this embodiment, the step of fixing preferably comprises wrapping the sheet around a tendon or ligament to be attached to bone. In another embodiment, when the connective tissue is a tendon, the attachment matrix is a gel that is injected around the tendon in tendon tunnels and between tendon and bone. Preferably, the gel is buffered.

In a preferred embodiment, the method of the present invention increases the biomechanical strength ofthe attachment of bone to connective tissue at least about 20%, and more preferably at least about 50%, and more preferably at least about 75%, and even more preferably at least about 100%, above the biomechanical strength of fixation of bone to connective tissue in the absence of the product.

### BRIEF DESCRIPTION OF THE DRAWINGS

Fig. 1 is a graph showing the fixation strength of grafts used for ACL reconstruction treated with Bone Protein as compared to controls at various time points.
Fig. 2 is a graph showing the fixation strength of grafts used for ACL reconstruction treated with Bone Protein as compared to controls treated with sponge alone at 8 weeks after treatment.

### DETAILED DESCRIPTION OF THE INVENTION

The present invention generally relates to a product and method for enhancing the attachment of connective tissue to bone, including repairing or regenerating such attachments where the attachment has been completely or partially severed as a result of an injury or surgical procedure, or has deteriorated due to a degenerative condition or disease, for example. The product and method of the present invention are useful for repairing any attachment of any connective tissue to bone, including, but not limited to, the attachment of tendon, ligaments and/or cementum to bone.

One embodiment of the present invention relates to a product for enhancing an attachment of connective tissue to bone. The product of the present invention includes: (a) a matrix configured to interface between connective tissue and bone; and, (b) a composition comprising a mixture of proteins associated with the matrix, such mixture being effective to induce the formation of a bone-cartilage-connective tissue interface at a site of attachment of connective tissue to bone. The mixture of proteins includes: transforming growth factor β1 (TGFβ1), bone morphogenetic protein (BMP)-2, BMP-3, and BMP-7. In one embodiment, the quantity of the TGFβ1 in the mixture is from about 0.01 % to about 10% of total proteins in the mixture; the quantity of the BMP-2 in the mixture is from about 0.01 % to about 10% of total proteins in the mixture; the quantity of the BMP-3 in the mixture is from about 0.1 % to about 15% of total proteins in the mixture; and, the quantity of the BMP-7 in the mixture is from about 0.01 % to about 10% of total proteins in the mixture. Unless otherwise specified, reference to percentages of proteins is based on weight to weight (w/w).

As discussed above, prior to the present invention, a variety of surgical procedures and/or compositions have been used and studied with regard to the reattachment and healing of tendon or ligament to bone. However, the compositions which have previously been used *in vivo* for tendon or ligament reattachment to bone have failed to reproduce the natural ontogeny of the connective tissue-to-bone attachment. Although the results of such studies have sometimes produced a *functional* attachment of tendon or ligament to bone, which may even include an attachment site having a progression from tendon or ligament to bone, the development of a physiologically natural connective tissue-to-bone attachment has not been demonstrated using such compositions. In the naturally occurring attachment of connective tissue to bone, as occurs in the developing vertebrate organism, there is a natural morphology of connective tissue to bone which includes a complex fan morphology and transition zones of fibrocartilage and calcified cartilage between the connective tissue and bone. See, for example, Benjamin et al., 1995, *supra.*

The present inventors believe that a more natural morphology of connective tissue to bone provides several benefits, including increased range of motion and strength, which, by way of example, would enhance the quality of life in patients who have undergone rotator cuff surgery. As another example, for ACL reconstruction applications, a more natural morphology of connective tissue to bone may decrease knee laxity. Decreased knee laxity is associated with decreased rates of osteoarthritis. Furthermore, for reconstructions which include both ACL and rotator cuff reconstructions, the product of the present invention may decrease the rate of surgical failures. Also, the proper biological tissue may more properly absorb physical stress and strain associated with normal musculoskeletal movement. In addition, the use of the product of the present invention is believed to enhance healing rates, which may decrease rehabilitation times. This advantage is particularly valuable, because it enables athletes to return to competition quickly and workers to return to work more quickly.

Physiological bone induction requires stimulatory and inhibitory factors for bone induction. A composition comprising only a stimulator of bone induction (e.g., a single recombinant BMP or multiple stimulators, as described by prior investigators) may sub-optimally regenerate tissue at the bone-tendon interface (or meniscus/other tissues). Because BP is a naturally derived mixture of proteins which contains both stimulators and inhibitors for bone induction, it induces more physiological like bone formation, as do the other compositions described herein, which have been developed based on the knowledge derived from BP. Physiological bone induction also includes another class of molecules which, when combined with other osteogenic or chondrogenic proteins such as those in BP, are chondrogenic inducers (TGFβ-1, TGFβ-2, TGFβ-3). TGFβ1-3 are found in BP. Although these proteins induce cartilage *in vitro,* they cannot induce cartilage (or bone) in the rodent subcutaneous assay (Wozney et al., 1993, Bone Morphogenetic proteins and their Gene Expression", *Cell Mol. Biol. Bone* 131-167). Subcutaneous induction of cartilage/bone is thought to be indicative of morphogenetic proteins since differentiation of pluripotent stem cells are required. *In vitro* chondrogenic activity may instead only increase matrix production of committed chondrogenic cells. The present invention describes a range of BMP/TGFβ mixtures that optimize physiological tendon-bone interface formation, and produce *in vivo* results which have not been observed using other compositions described prior to the present invention.

The phrase "natural ontogeny of the attachment of connective tissue-to-bone", and similar such phrases, are therefore used herein to refer to such development of the connective tissue-to-bone attachment that naturally occurs in an organism, as during embryogenesis. Prior to the present invention, compositions used to enhance the reattachment or repair of connective tissue to bone have failed to induce the formation of such a natural morphology, and in particular, a preferred fibrocartilage and calcified cartilage transition zone.

In contrast to previous studies, the present inventors have discovered a product for the enhancement of a connective tissue-to-bone attachment that includes a complex mixture of proteins from Bone Protein (BP), BP being described in detail below. The present inventors have found that the product of the present invention, in contrast to previously described compositions for use in a connective tissue-to-bone attachment, can recapitulate natural connective tissue-to-bone ontogeny. Specifically, the product of the present invention is effective to induce the formation of a bone-*fibrocartilage*/*calcified cartilage*-connective tissue interface, also referred to herein generally as a bone-cartilage-connective tissue interface, at a site of attachment of connective tissue to bone (i.e., a physiologically natural tissue type at the site). Moreover, the product induces a greater quantity ofbone and the bone appears faster and in closer proximity to the connective tissue. This physiologically accurate healing increases the fixation strength at earlier time points, and can specifically increase fixation strength in patients with degenerative tendon and/or bone pathology. Without being bound by theory, the present inventors believe that the repair of connective tissue-to-bone attachments, and particularly where the natural tissue is completely destroyed or deteriorated, requires the maintenance of a sufficient concentration of a particular complex mixture of repair factors at the site for a time sufficient to induce a proper formation of the attachment which faithfully mimics the natural ontogeny, this natural ontogeny being based on a very complicated system of different factors, factor combinations and factor concentrations with temporal and local gradients. A single recombinant factor or a few recombinant factors may lack the inductive complexity to mimic the natural ontogeny of the attachment to a sufficient degree. Similarly, the system used to provide one or a few recombinant factors may not have been able to mimic the gradient complexity of the natural system to a satisfactory degree or to maintain a factor concentration for a time that is sufficient to allow a full and permanent differentiation of precursor cells to the proper cell type in the proper location. Therefore, the present inventors have discovered a product and process which produces a quantitative and qualitative result superior to any previously described for connective tissue-to-bone attachment.

It is recognized that the term "connective tissue" is understood in the art to refer to the tissue which binds together and is the support of the various structures of the body, and which is made up of fibroblasts, fibroglia, collagen fibrils, and elastic fibrils. It is derived from the mesoderm and in a broad sense includes the collagenous, elastic, mucous, reticular, osseous and cartilaginous tissues (Dorland's Illustrated Medical Dictionary, 27^{th} Edition, 1988, W.B. Saunders Company). For convenience in discussion of the embodiments of the present invention, however, the term "connective tissue" as used herein refers to connective tissue which connects to bone, such as connective tissue that connects bone or cartilage to bone or muscle to bone, and particularly is selected from the group of tendon and ligament. In one embodiment, the term "connective tissue" is used to refer to cementum, which is defined as the thin layer of calcified tissue that covers the dentin of the root of a tooth and anchors the tooth in its bony socket. According to the present invention, the terms "ligament" and "tendon" are defined according to the definition in Dorland's Illustrated Medical Dictionary, *ibid.* Specifically, the term "ligament" is defined as a band of fibrous connective tissue that connects bones to cartilage, serving to support and strengthen joints. The term "tendon" is defined as a fibrous cord of connective tissue in which the fibers of a muscle end and by which the muscle is attached to a bone or other structure.

The composition ofthe present invention provides a mixture of attachment-enhancing proteins. According to the present invention, the term "enhancing", particularly with regard to enhancing an attachment of connective tissue to bone, refers to any measure of augmenting, amplifying, improving, increasing, or supplementing the attachment of connective tissue to bone such that the attachment forms in a manner that more closely mimics the natural ontogeny of connective tissue-to-bone attachment, as compared to the attachment of connective tissue to bone that would occur in the absence of the product, or in the absence of the composition portion of the product. More particularly, a measurable enhancement of the rate of attachment of connective tissue to bone is any measurable improvement in the time between Day 0 of the attachment (i.e., the day the product is implanted into the patient) and the day on which it is determined that a suitable bone-cartilage-connective tissue interface has developed at the site of attachment as compared to a substantially similar attachment of connective tissue to bone that has been performed in the absence of the product of the present invention or in the absence of the composition portion of the product ofthe present invention. Development of a suitable bone-cartilage-connective tissue interface is defined as an initial indication of better biomechanical strength of the attachment. In a preferred embodiment, the product of the present invention measurably enhances the rate of attachment of connective tissue to bone, as compared to a rate of attachment of connective tissue to bone that occurs in the absence of the product of the present invention or in the absence of the composition portion of the product, by at least 20%, and more preferably, by at least about 50%, and more preferably by at least about 100%.

In addition, the use of the attachment product of the present invention to attach connective tissue to bone results in a measurable enhancement of the quality of attachment of the connective tissue to bone as compared to the quality of attachment that occurs in the absence of the product of the present invention or in the absence of the composition portion of the product of the present invention. A measurable enhancement in the quality of the attachment of the connective tissue to bone is defined as any measurable improvement in quality of attachment and particularly, in the formation of a bone-cartilage-connective tissue interface at the site of attachment, with an improvement being defined as development of a more natural ontogeny ofthe attachment of connective tissue-to-bone, which can be indicated by complex fan morphology and transition zones of fibrocartilage and calcified cartilage between the connective tissue and bone, induction of cellular infiltration into the product, induction of cellular proliferation, and production of cellular and spatial organization to form a three-dimensional tissue that more nearly represents endogenous connective tissue to bone attachment.

Various embodiments of the composition of the present invention are described in detail below. For general reference, however, the following description is provided. Proteins that can be included in the mixture of proteins in a composition according to the present invention, also referred to herein as "attachment-enhancing proteins", are anyproteins which, alone and/or in combination with other proteins, are effective to enhance the attachment of connective tissue to bone. Examples of attachment-enhancing proteins include, but are not limited to, TGFβ superfamily members, growth factors, bone matrix proteins, and serum proteins.

As used herein, a "TGFβ superfamily protein" can be any protein of the art-recognized superfamily of extracellular signal transduction proteins that are structurally related to TGFβ 1-5 and includes functional equivalents of such naturally occurring proteins, such as fragments of such proteins which retain the biological activity of the naturally occurring protein. According to the present invention, the mixture of proteins in the composition of the present invention typically includes at least two different members of the TGFβ superfamily proteins. In other embodiments, the mixture of proteins include at least three different members of the TGFβ superfamily proteins, and in alternative embodiments, at least four, five, six, seven, eight, nine, and most preferably ten different members of the TGFβ superfamily proteins. Preferably, a TGFβ superfamily protein suitable for use in the present invention is selected from the following proteins: TGFβ1, TGFβ2, TGFβ3, bone morphogenetic protein (BMP)-2, BMP-3, BMP-4, BMP-5, BMP-6, BMP-7, BMP-8, BMP-9, cartilage-derived morphogenetic protein (CDMP)-1 (BMP-12), CDMP-2 (BMP-13), and/or CDMP-3 (BMP-14). More preferably, the TGFβ superfamily protein is selected from the group of: TGFβ1, TGFβ2, TGFβ3, BMP-2, BMP-3, BMP-4, BMP-5, BMP-6, BMP-7, CDMP-1, CDMP-2, and/or CDMP-3. In one embodiment, the TGFβ superfamily proteins in a Bone Protein composition of the present invention include at least one of BMP2-8, and more preferably, at least one of TGFβ1-5, and even more preferably, at least one of CDMP 1-3. In a preferred embodiment, the mixture of proteins includes at least TGFβ1, BMP-2, BMP-3, and BMP-7. In another preferred embodiment, the mixture of proteins includes at least one TGFβ protein (and most preferably TGFβ1), BMP-2, BMP-3, and BMP-7, and at least one of CDMP1-3.

In some aspects ofthe present invention, the composition ofthe present invention can include at least one bone matrix protein and/or at least one growth factor protein. In a preferred aspect, the mixture ofproteins includes at least one bone matrix protein and at least one growth factor protein. In a more preferred embodiment, the attachment-enhancing proteins include, in increasing preference, at least two, three, four, and most preferably five different bone matrix proteins, and/or at least two growth factor proteins.

As used herein, "bone matrix proteins" are any of a group of proteins known in the art to be a component of or associated with the minute collagenous fibers and ground substances which form bone matrix, and includes functional equivalents of such naturally occurring proteins, such as fragments of such proteins which retain the biological activity of the naturally occurring protein. As used herein, a bone matrix protein is not a member of the TGFβ superfamily as described herein, nor a growth factor protein as described herein. Bone matrix proteins can include, but are not limited to, osteocalcin, osteonectin, bone sialoprotein (BSP), lysyloxidase, cathepsin L pre, osteopontin, matrix GLA protein (MGP), biglycan, decorin, proteoglycan-chondroitin sulfate III (PG-CS III), bone acidic glycoprotein (BAG-75), thrombospondin (TSP) and/or fibronectin. Preferably, bone matrix proteins which are in a composition of the present invention include one or more of: osteocalcin, osteonectin, MGP, TSP, B SP, lysyloxidase and cathepsin L pre. In one embodiment, bone matrix proteins include osteocalcin, osteonectin, BSP, lysyloxidase and cathepsin L pre.

As used herein, "growth factor proteins" are any of a group of proteins characterized as an extracellular polypeptide signaling molecule that stimulates a cell to grow or proliferate, and includes functional equivalents of such naturally occurring proteins, such as fragments of such proteins which retain the biological activity of the naturally occurring protein. Such growth factors may also have other actions besides the induction of cell growth or proliferation. As used herein, a growth factor is not a member of the TGFβ superfamily as defmed herein nor is it a bone matrix protein as defmed herein. Preferably, growth factor proteins suitable for use with the product of the present invention include one or more of: fibroblast growth factor I (FGF-I), FGF-II, FGF-9, leukocyte inhibitory factor (LIF), insulin, insulin growth factor I (IGF-I), IGF-II, platelet-derived growth factor AA (PDGF-AA), PDGF-BB, PDGF-AB, stromal derived factor-2 (SDF-2), pituitary thyroid hormone (PTH), growth hormone, hepatocyte growth factor (HGF), epithelial growth factor (EGF), transforming growth factor-α (TGFα) and hedgehog proteins. A most preferred growth factor protein for use with the product of the present invention is FGF-I.

In one embodiment of the present invention, a composition including a mixture of attachment-enhancing proteins can include one or more serum proteins. As used herein, serum proteins are any of a group ofproteins that is known to be a component of serum, and includes functional equivalents of such naturally occurring components, such as fragments of such proteins which retain the biological activity of the naturally occurring protein. A serum protein is not a member of the TGFβ superfamily, a bone matrix protein or a growth factor, as described herein. Preferably, the attachment-enhancing proteins include, in increasing preference, at least one, two, three, and most preferably four different serum proteins. Serum proteins suitable for use with the product of the present invention include one or more of albumin, transferrin, α2-Hs GlycoP, IgG, α1-antitrypsin, β2-microglobulin, Apo A1 lipoprotein (LP) and Factor XIIIb. Preferably, serum proteins suitable for use with the product of the present invention include one or more of albumin, transferrin, Apo A1 LP and Factor XIIIb.

In one embodiment, the percentage of TGFβ superfamily members within the mixture ranges between about 0.1 % to about 100% of the total mixture, and preferably between about 0.5% to about 100% of the total mixture, and more preferably between about 0.1 % to about 50% of the total mixture, and more preferably between about 0.5% to about 50% of the total mixture, and more preferably, between about 0.5% and about 25%, and even more preferably, between about 1% and about 10% of the total mixture. The percentage of growth factors within the mixture ranges between about 0.01% to about 50% of the total mixture, and preferably, between about 0.05% and about 25%, and even more preferably, between about 0.1% and about 10% of the total mixture. The percentage of serum and bone matrix protein components, either separately or combined, ranges between about 20% to about 98%, and preferably between about 40% to about 98%, and even more preferably between about 80% to about 98%. Unless otherwise specified, reference to percentages of proteins or ratios of proteins either to the mixture of proteins or to specific proteins is based on weight to weight (w/w).

Having generally discussed certain aspects of mixtures of proteins which may be present in a composition of the present invention, particular preferred embodiments of such a composition are described below.

In a first embodiment of the present invention, a composition of the present invention includes a mixture of proteins which includes: transforming growth factor β1 (TGFβ1), bone morphogenetic protein (BMP)-2, BMP-3, and BMP-7. The quantity of the TGFβ1 in the mixture is typically from about 0.01 % to about 10% of total proteins in the mixture, or from about 0.01 to about 1%, or from about 0.01 to about 0.5%, or from about 0.01% to about 0.1%, or from about 0.05 to about 1%, or from about 0.05 to about 10%, or from about 0.1 to about 1% or from about 0.1 to about 10% of total proteins. In one embodiment, the ratio of TGFβ1 to all other proteins in the mixture is up to about 1:10. The quantity of the BMP-2 in the mixture is typically from about 0.01% to about 10% of total proteins in the mixture, or from about 0.01 to about 1%, or from about 0.01 to about 0.1%, or from about 0.1 to about 1%, or from about 0.1 to about 10% of total proteins, and in one embodiment, is present in a quantity of from about 0.1% to about 5% of total proteins in the mixture. The quantity of the BMP-3 in the mixture is typically from about 0.1 % to about 15% of total proteins in the mixture, or from about 0.1 to about 1 %, or from about 0.01 to about 15%, or from about 0.01 to about 1%, or from about 0.01 to about 0.1%, and in one embodiment, is present in a quantity of from about 0.1 % to about 10% of total proteins in the mixture. The quantity of the BMP-7 in the mixture is typically from about 0.01 % to about 10% of total proteins in the mixture, or from about 0.01 to about 1%, or from about 0.01 to about 0.1%, or from about 0.1 to about 1%, or from about 0.1 to about 10% of total proteins, and in one embodiment, is present in a quantity of from about 0.1 % to about 5% of total proteins in the mixture. The amino acid and nucleic acid sequence for each of the above-referenced proteins are known in the art and can be publicly accessed, for example, through a database such as GenBank. Additionally, these proteins can be purified, if desired, from an appropriate source, such as demineralized bone. For example, high purity TGFβ-1 can be isolated from bovine bone using methods disclosed by Seyedin (Ogawa et al., Meth. Enzymol., 198:317-327 (1991); Seyedin et al., PNAS, 82:2267-71 (1985)). Additionally, bone-derived mixtures of proteins containing these proteins in the recited ranges can be prepared from demineralized bone using procedures known in the art and discussed in some detail herein.

In one aspect of this embodiment of the present invention, the mixture of proteins comprises TGFβ superfamily proteins: TGFβ1, bone morphogenetic protein (BMP)-2, BMP-3, and BMP-7, wherein the TGFβ superfamily proteins together comprise from about 0.5% to about 100% of the mixture of proteins. Alternatively, the TGFβ superfamily proteins can be present at a percentage of from about 0.5% to about 50%, or from about 0.5% to about 25% of the mixture of proteins, or from about 1% to about 10% of the mixture of proteins.

In one aspect of this embodiment of the present invention, the mixture of proteins further includes one or more of the following TGFβ superfamily proteins: TGFβ2, TGFβ3, BMP-4, BMP-5, BMP-6, BMP-8, BMP-9, and cartilage-derived morphogenetic protein (CDMP), which can include one or more of CDMP-1 (i.e., BMP-12), CDMP-2 (i.e., BMP-13) or CDMP-3 (i.e., BMP-14). The quantity of TGFβ2 in such a mixture is typically from about 0.5% to about 12% of the total mixture of proteins, although additional TGFβ2 can be added, if desired, to increase the effectiveness of the composition to enhance the attachment of connective tissue to bone, up to about 20% of the total mixture. The quantity of TGFβ3 in such a mixture is typically from about 0.01 % to about 15% of the total mixture of proteins although additional TGFβ3 can be added, if desired, to increase the effectiveness of the composition to enhance the attachment of connective tissue to bone, up to about 25% of the total mixture. The quantity of BMP-4 in such a mixture typically comprises from about 0.01 % to about 1% of the total mixture of proteins, although additional BMP-4 can be added to enhance the attachment of connective tissue to bone, if desired, up to as much as about 10% of the total mixture of proteins. The quantity of BMP-5 in the mixture of proteins is typically from about 0.01 % to about 1% of the total mixture of proteins, although additional BMP-5 can be added to enhance the attachment of connective tissue to bone, if desired, up to as much as about 10% of the total mixture of proteins. The quantity of BMP-6 in the mixture of proteins is typically from about 0.01% to about 1% of the total mixture of proteins, although additional BMP-6 can be added to enhance the attachment of connective tissue to bone, if desired, up to as much as about 10% of the total mixture of proteins. The quantity of CDMPs in the mixture of proteins is typically from about 0.01 % to about 1% of the total mixture of proteins, although additional CDMPs can be added to enhance the attachment of connective tissue to bone, if desired, up to as much as about 10% of the total mixture of proteins.

In one aspect of this embodiment, the mixture of proteins can additionally include at least one bone matrix protein. Bone matrix proteins, including preferred bone matrix proteins for use in the composition of the present invention, are generally described above. The bone matrix proteins are typically present in the mixture in a quantity from about 20% to about 98% of the total mixture of proteins, although the quantity of bone matrix proteins can be less than 20%, if desired. In one embodiment, the bone matrix proteins are present in the mixture in a quantity from about 40% to about 98% of the total mixture of proteins.

In another aspect of this embodiment, the mixture ofproteins can additionally include at least one growth factor protein. Growth factor proteins are generally described above, including preferred growth factor proteins for use in the composition ofthe present invention. Typically, the growth factor protein is present in the mixture of proteins at a quantity from about 0.01% to about 50% of the total mixture of proteins. In other embodiments, the quantity of growth factor proteins in the mixture is from about 0.5 % to about 25% of the total mixture of proteins; or from about 0.1 % to about 10% of the total mixture of proteins. When the growth factor is FGF-I, the quantity of FGF-I in the mixture of proteins is typically from about 0.001% to about 10% of the total mixture of proteins.

In yet another aspect of this embodiment, the mixture of proteins can include one or more serum proteins. Serum proteins have been generally described above, including preferred serum proteins for use in a composition of the present invention. Serum proteins are typically present in the mixture of proteins in a quantity from about 20% to about 98% of the total mixture ofproteins, although the quantity of serum proteins can be less than 20%, if desired.. In one embodiment, the serum proteins are present in the mixture in a quantity from about 40% to about 98% of the total mixture of proteins.

In one aspect of this embodiment of the present invention, a mixture of proteins suitable for use in a composition portion of a product of the present invention includes the following proteins: TGFβ1, TGFβ2, TGFβ3, BMP-2, BMP-3, BMP-4, BMP-5, BMP-6, BMP-7, CDMP, FGF-I, osteocalcin, osteonectin, BSP, lysyloxidase, cathepsin L pre, albumin, transferrin, Apo A1 LP and Factor XIIIb. In yet another embodiment, a suitable mixture of proteins includes the mixture of proteins referred to herein as Bone Protein (BP), which is defined herein as a partially-purified protein mixture from bovine long bones as described in Poser and Benedict, WO 95/13767, incorporated herein by reference in its entirety.

In another aspect of this embodiment of the present invention, the composition of the present invention has one or more of the following identifying characteristics: (1) an ability to induce cellular infiltration at the site of attachment of connective tissue to bone; (2) an ability to induce cellular proliferation at the site of attachment of connective tissue to bone; (3) an ability to induce formation of bone at the site of attachment of connective tissue to bone; (4) an ability to induce the formation of a connective tissue selected from tendon and/or ligament at the site of attachment of connective tissue to bone; and, (5) an ability to induce the formation of fibrocartilage and calcified cartilage at the site of attachment of connective tissue to bone. In a preferred embodiment, the composition preferably does not substantially induce bone resorption at the site of attachment.

In yet another aspect of this embodiment of the present invention, the mixture of proteins, when used at a concentration of at least about 10 µg per 6.5-7.3 mg of bovine tendon collagen in a rat subcutaneous assay, induces a bone score of at least about 1.0, and more preferably at least about 1.25, and more preferably at least about 1.5, and more preferably at least about 1.75, and even more preferably at least about 2.0, using a bone grading scale set forth in Table 1 (Examples 1 and 6), and induces a cartilage score of at least about 1.0, and more preferably at least about 1.25, and more preferably at least about 1.5, and more preferably at least about 1.75, and even more preferably at least about 2.0, using a cartilage grading scale set forth in Table 8 (Example 6). A rat subcutaneous assay suitable for determining bone and cartilage scores according to this aspect, and the grading scales of Tables 1 and 8 are described in detail in the Examples Section.

In one embodiment of the present invention, a suitable composition for use in the present invention can be characterized by its ability, when combined with an attachment matrix (e.g., a collagen sponge) and used in a rat subcutaneous assay as described in Example 6, to induce an ordered ring of bone formation around the periphery of the collagen sponge. More particularly, a suitable composition for use in the present invention, when used in a rat subcutaneous assay as described in Example 6, produces an ordered ring on the outside of the collagen sponge and just interior to this ring, produces an ordered cartilage ring. Without being bound by theory, the present inventors believe that this characteristic is indicative of compositions which will provide a superior tendon to bone fixation.

In another embodiment of the present invention, a composition includes a mixture of proteins which includes a bone-derived formulation with chondrogenic and osteogenic activity. Preferably, a composition comprising such a bone-derived formulation has one or more characteristics which include: (1) an ability to induce cellular infiltration at the site of attachment of connective tissue to bone; (2) an ability to induce cellular proliferation at the site of attachment of connective tissue to bone; (3) an ability to induce formation of bone at the site of attachment of connective tissue to bone; (4) an ability to induce the formation of a connective tissue selected from tendon and/or ligament at the site of attachment of connective tissue to bone; and, (5) an ability to induce the formation of fibrocartilage and calcified cartilage at the site of attachment of connective tissue to bone. In a preferred embodiment, a composition comprising a bone-derived formulation preferably does not substantially induce bone resorption at the site of attachment. In one embodiment, a bone-derived composition, when used at a concentration of at least about 10 µg per 6.5-7.3 mg of bovine tendon collagen in a rat subcutaneous assay, induces a bone score of at least about 1.0, and more preferably at least about 1.25, and more preferably at least about 1.5, and more preferably at least about 1.75, and even more preferably at least about 2.0, using a bone grading scale set forth in Table 1 (Examples 1 and 6), and induces a cartilage score of at least about 1.0, and more preferably at least about 1.25, and more preferably at least about 1.5, and more preferably at least about 1.75, and even more preferably at least about 2.0, using a cartilage grading scale set forth in Table 8 (Example 6). In another embodiment of the present invention, a composition comprising a bone-derived formulation is characterized by its ability, when combined with an attachment matrix (e.g., a collagen sponge) and used in a rat subcutaneous assay as described in Example 6, to induce an ordered ring of bone formation around the periphery of the collagen sponge. More particularly, a suitable composition for use in the present invention, when used in a rat subcutaneous assay as described in Example 6, produces an ordered ring on the outside of the collagen sponge and just interior to this ring, produces an ordered cartilage ring.

According to the present invention, a "bone-derived formulation having osteogenic and chondrogenic activity" refers to any mixture of proteins containing a complex mixture of proteins which is isolated, or derived, (e.g., by at least one, and typically, multiple purification steps) from a starting material of bone, and which is osteogenic and chondrogenic *in vivo.* Preferably, the bone-derived formulation is capable of inducing bone and cartilage formation in an *in vivo* rat subcutaneous assay such as that described in the Examples section of the Rosen modified Sampath-Reddi rodent assay (Sampath et al., Proc. Nat'l Acad. Sci. USA, 80(21):6591-5 (1983)). Particularly preferred bone-derived formulations for use in the present invention include Bone Protein (BP) and subfractions and related derivatives thereof. The Examples Section describes examples of BP (Examples 5 and 6), subfractions thereof (Examples 5 and 8) and related derivatives thereof (Example 7).

The starting material of bone can be a bone sample from any source, including, but not limited to, bovine bone and human bone. The bone can be processed using any of a number of methods known in the art for producing compositions which have osteogenic and/or chondrogenic activity (See for example, U.S. Patent No. 4,563,489 to Urist; U.S. Patent No. 5,629,009 to Laurencin et al.; PCT Publication No. WO 92/09697 to Bentz et al.; and Poser and Benedict, PCT Publication No. W095/13767), and refined by additional processing steps, as necessary, to improve the ability of such composition to enhance the attachment of connective tissue to bone (e.g., further purification to increase relative levels of select proteins). According to the present invention, a "bone-derived formulation" is not used to refer to mixtures of one or more recombinant proteins, since recombinant proteins are not produced using bone as a starting material.

For example, one method for producing Bone Protein according to the present invention, and as described, for example, in U.S. Patent No. 5,290,763, entitled "Osteoinductive Protein Mixtures and Purification Processes", incorporated herein by reference in its entirety, typically includes the steps of conducting anion exchange chromatography on a demineralized bone extract solution, a cation exchange procedure, and reverse phase HPLC procedure.

According to the present invention, proteins can be added to the composition of the present invention, and particularly, to a bone-derived composition, as additional exogenous proteins. An "exogenous protein" refers to a protein that is in substantially pure form and which is not a part of a bone-derived osteogenic or chondrogenic formulation of proteins, for example (i.e., the exogenous protein was not isolated with the bone-derived osteogenic or chondrogenic formulation of proteins). The exogenous protein is instead added to the formulation as an additional protein from a different source. It is to be understood that the bone-derived osteogenic or chondrogenic formulation of proteins can naturally contain the same proteins (i.e., "endogenous" proteins) which can additionally be added as an exogenous source. However, addition of an exogenous protein is intended as a means of increasing the total amount ofa particular protein in the composition beyond what is naturally found in bone and mixtures derived therefrom. The exogenous protein can be a recombinant protein or a substantially purified protein from any suitable source, such as bone.

In one embodiment of the present invention, each of the attachment-enhancing proteins in the composition is provided by the composition either: (1) directly as a protein that is associated with the matrix, or (2) as a recombinant nucleic acid molecule associated with the matrix, such recombinant nucleic acid molecule encoding the protein and being operatively linked to a transcription control sequence such that the protein can be expressed under suitable conditions. Therefore, a composition of the present invention can include proteins, recombinant nucleic acid molecules, or a combination of proteins and recombinant nucleic acid molecules, such composition providing the attachment-enhancing proteins described above.

According to the present invention, an attachment-enhancing protein can be obtained from its natural source, produced using recombinant DNA technology, or synthesized chemically. As used herein, an attachment-enhancing protein can be a full-length protein (i.e., in its full-length, naturally occurring form), any homologue of such a protein, any fusion protein containing such a protein, or any mimetope of such a protein. The amino acid sequences for attachment-enhancing proteins disclosed herein, as well as nucleic acid sequences encoding the same are known in the art and are publicly available, for example, from sequence databases such as GenBank. Such sequences can therefore be obtained and used to produce proteins and recombinant nucleic acid molecules of the present invention.

A homologue is defined as a protein in which amino acids have been deleted (e.g., a truncated version of the protein, such as a peptide or fragment), inserted, inverted, substituted and/or derivatized (e.g., by glycosylation, phosphorylation, acetylation, myristoylation, prenylation, palmitation, amidation and/or addition of glycosylphosphatidyl inositol). A homologue of an attachment-enhancing protein is a protein having an amino acid sequence that is sufficiently similar to a naturally occurring attachment-enhancing protein amino acid sequence that the homologue has substantially the same or enhanced biological activity compared to the corresponding naturally occurring protein. Protein homologues can be the result of natural allelic variation or natural mutation. In one embodiment, an attachment-enhancing homologue comprises an amino acid sequence comprising at least about 6, and more preferably at least about 12 and more preferably at least about 24 contiguous amino acid residues of an amino acid sequence of a naturally occurring (i.e., wild-type) attachment-enhancing protein. In another embodiment, an attachment-enhancing homologue is encoded by a nucleic acid sequence comprising at least about 18, and more preferably at least about 36, and even more preferably at least about 72 contiguous nucleotides of a nucleic acid sequence encoding a naturally occurring attachment-enhancing protein.

As used herein, a mimetope (also referred to as a synthetic mimic) of an attachment-enhancing protein according to the present invention refers to any compound that is able to mimic the activity of a naturally occurring attachment-enhancing protein, often because the mimetope has a structure that mimics the attachment-enhancing protein. In one embodiment, a mimetope is identified as being capable of being bound by an antibody that selectively binds to the naturally occurring protein on which the mimetope is based. Mimetopes can be, but are not limited to: peptides that have been modified to decrease their susceptibility to degradation; anti-idiotypic and/or catalytic antibodies, or fragments thereof; non-proteinaceous immunogenic portions of an isolated protein (e.g., carbohydrate structures); and synthetic or natural organic molecules, including nucleic acids. Such mimetopes can be designed using computer-generated structures of naturally occurring attachment-enhancing protein. Mimetopes can also be obtained by generating random samples of molecules, such as oligonucleotides, peptides or other organic or inorganic molecules, and screening such samples by affinity chromatography techniques using the corresponding binding partner. Mimetopes can be designed using computer-generated three dimensional structures of attachment-enhancing proteins, for example. Mimetopes can also be obtained by generating random samples of molecules, such as oligonucleotides, peptides or other organic or inorganic molecules, and screening such samples for attachment-enhancing protein biological activity or affinity for a binding partner of a naturally occurring attachment-enhancing protein.

According to the present invention, the peptide and synthetic mimetopes of proteins can be designed by creating a new chemical or biological (e.g. protein, peptide, antibody, antisense, ribozyme) compound or searching databases of libraries of known compounds (e.g., a compound listed in a computational screening database containing three dimensional structures of known compounds). Designing can also be performed by simulating chemical or biological compounds having substitute moieties at certain structural features. The step of designing can include selecting a compound based on a known function of the compound. A preferred step of designing comprises computational screening of one or more databases of compounds in which the three dimensional structure of the compound is known and is interacted (e.g., docked, aligned, matched, interfaced) with the three dimensional structure (or predicted three dimensional structure) of the protein by computer (e.g. as described by Humblet and Dunbar, Animal Reports in Medicinal Chemistry, vol. 28, pp. 275-283, 1993, M Venuti, ed., Academic Press). Methods to synthesize suitable chemical or biological compounds are known to those of skill in the art and depend upon the structure of the chemical or other molecule being synthesized. Methods to evaluate the bioactivity of the synthesized compound depend upon the bioactivity of the compound (e.g., inhibitory or stimulatory) and are disclosed herein.

Various methods of drug design are disclosed in Maulik et al., 1997, Molecular Biotechnology: Therapeutic Applications and Strategies, Wiley-Liss, Inc., which is incorporated herein by reference in its entirety. Maulik et al. disclose, for example, methods of directed design, in which the user directs the process of creating novel molecules from a fragment library of appropriately selected fragments; random design, in which the user uses a genetic or other algorithm to randomly mutate fragments and their combinations while simultaneously applying a selection criterion to evaluate the fitness of candidate ligands; and a grid-based approach in which the user calculates the interaction energy between three dimensional receptor structures and small fragment probes, followed by linking together of favorable probe sites.

In a method of drug design, it is not always necessary to align a candidate compound (i.e., a compound being analyzed in, for example, a computational screening method) to each residue in a target site. Suitable candidate compounds can align to a subset of residues described for a target site. Preferably, a candidate compound comprises a conformation that promotes the formation of covalent or noncovalent crosslinking between the target site and the candidate compound. Preferably, a candidate compound binds to a surface adjacent to a target site to provide an additional site of interaction in a complex. It will be appreciated by one of skill in the art that it is not necessary that the complementarity between a candidate compound and a target site extend over all residues specified here in order to inhibit or promote binding of a ligand.

In general, the design of a chemical or biological compound possessing stereochemical complementarity can be accomplished by means of techniques that optimize, chemically or geometrically, the "fit" between a compound and a target site. Such techniques are disclosed by, for example, Sheridan and Venkataraghavan, Acc. Chem Res., vol. 20, p. 322, 1987: Goodford, J. Med. Chem., vol. 27, p. 557, 1984; Beddell, Chem. Soc. Reviews, vol. 279,1985; Hol, Angew. Chem., vol. 25, p. 767,1986; and Verlinde and Hol, Structure, vol. 2, p. 577, 1994, each of which are incorporated by this reference herein in their entirety.

According to the present invention, a fusion protein is a protein that includes an attachment-enhancing protein-containing domain attached to one or more fusion segments. Suitable fusion segments for use with the present invention include, but are not limited to, segments that can: enhance a protein's stability; enhance the biological activity of an attachment-enhancing protein; and/or assist purification of an attachment-enhancing protein (e.g., by affinity chromatography). A suitable fusion segment can be a domain of any size that has the desired function (e.g., imparts increased stability, imparts enhanced biological activity to a protein, and/or simplifies purification of a protein). Fusion segments can be joined to amino and/or carboxyl termini of the attachment-enhancing protein-containing domain of the protein and can be susceptible to cleavage in order to enable straight-forward recovery of an attachment-enhancing protein. Fusion proteins are preferably produced by culturing a recombinant cell transformed with a fusion nucleic acid molecule that encodes a protein including the fusion segment attached to either the carboxyl and/or amino terminal end of an attachment-enhancing protein-containing domain. Preferred fusion segments include a metal binding domain (e.g., a poly-histidine segment); an immunoglobulin binding domain (e.g., Protein a; Protein G; T cell; B cell; Fc receptor or complement protein antibody-binding domains); a sugar binding domain (e.g., a maltose binding domain); streptavidin, avidin, biotin, and/or a "tag" domain (e.g., at least a portion of β-galactosidase, a strep tag peptide, other domains that can be purified using compounds that bind to the domain, such as monoclonal antibodies).

As discussed above, one or more of the attachment-enhancing proteins in the composition can be provided by the composition as a recombinant nucleic acid molecule associated with the attachment matrix, such recombinant nucleic acid molecule encoding an attachment-enhancing protein and being operatively linked to a transcription control sequence such that the protein can be expressed under suitable conditions. A recombinant nucleic acid molecule useful in the present invention can include an isolated nucleic acid molecule encoding a naturally occurring attachment-enhancing protein or a homologue of such a nucleic acid molecule, the latter of which is described in more detail below. A nucleic acid molecule useful in the present invention can include one or more regulatory regions, full-length or partial coding regions, or combinations thereof.

In accordance with the present invention, an isolated nucleic acid molecule is a nucleic acid molecule that has been removed from its natural milieu (i.e., that has been subject to human manipulation) and can include DNA, RNA, or derivatives of either DNA or RNA. As such, "isolated" does not reflect the extent to which the nucleic acid molecule has been purified. An isolated nucleic acid molecule encoding an attachment-enhancing protein can be isolated from its natural source or produced using recombinant DNA technology (e.g., polymerase chain reaction (PCR) amplification, cloning) or chemical synthesis. Isolated nucleic acid molecules can include, for example, natural allelic variants and nucleic acid molecule homologues modified by nucleotide insertions, deletions, substitutions, and/or inversions in a manner such that the modifications do not substantially interfere with the nucleic acid molecule's ability to encode an attachment-enhancing protein of the present invention or to form stable hybrids under stringent conditions with natural gene isolates. An isolated nucleic acid molecule can include degeneracies. As used herein, nucleotide degeneracies refers to the phenomenon that one amino acid can be encoded by different nucleotide codons. Thus, the nucleic acid sequence of a nucleic acid molecule that encodes an attachment-enhancing protein of the present invention can vary due to degeneracies.

A nucleic acid molecule homologue can be produced using a number of methods known to those skilled in the art (see, for example, Sambrook et al., *ibid.*). For example, nucleic acid molecules can be modified using a variety of techniques including, but not limited to, by classic mutagenesis and recombinant DNA techniques (e.g., site-directed mutagenesis, chemical treatment, restriction enzyme cleavage, ligation of nucleic acid fragments and/or PCR amplification), or synthesis of oligonucleotide mixtures and ligation of mixture groups to "build" a mixture of nucleic acid molecules and combinations thereof. Nucleic acid molecule homologues can be selected by hybridization with a naturally occurring gene or by screening for the function of a protein encoded by the naturally occurring nucleic acid molecule. Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding an attachment-enhancing protein.

Knowing the nucleic acid sequence encoding a naturally occurring attachment-enhancing protein according to the present invention allows one skilled in the art to, for example, (a) make copies of those nucleic acid molecules, and (b) obtain nucleic acid molecules including at least a portion of such nucleic acid molecules (e.g., nucleic acid molecules including full-length genes, full-length coding regions, regulatory control sequences, truncated coding regions). Such nucleic acid molecules can be obtained in a variety of ways including screening appropriate expression libraries with antibodies; traditional cloning techniques using oligonucleotide probes to screen appropriate libraries or DNA; and PCR amplification of appropriate libraries or DNA using oligonucleotide primers. Techniques to clone and amplify genes are disclosed, for example, in Sambrook et al., *ibid.*

According to the present invention, a nucleic acid molecule encoding an attachment-enhancing protein is operatively linked to one or more transcription control sequences to form a recombinant molecule. The phrase "operatively linked" refers to linking a nucleic acid molecule to a transcription control sequence in a manner such that the molecule is able to be expressed when transfected (i.e., transformed, transduced or transfected) into a host cell. A recombinant nucleic acid molecule includes a recombinant vector, which is any nucleic acid sequence, typically a heterologous sequence, which is operatively linked to the isolated nucleic acid molecule encoding the attachment-enhancing protein, which is capable of enabling recombinant production of the protein, and which is capable of delivering the nucleic acid molecule into a host cell according to the present invention. Such a vector can contain nucleic acid sequences that are not naturally found adjacent to the isolated nucleic acid molecules to be inserted into the vector. The vector can be either RNA or DNA, either prokaryotic or eukaryotic, and preferably in the present invention, is a virus or a plasmid.. Recombinant vectors can be used in the cloning, sequencing, and/or otherwise manipulating of nucleic acid molecules. Recombinant vectors are preferably used in the expression of nucleic acid molecules, and can also be referred to as expression vectors. Preferred recombinant vectors are capable of being expressed in a transfected host cell, and particularly, in a transfected mammalian host cell *in vivo.*

One type of recombinant vector useful in a recombinant nucleic acid molecule of the present invention is a recombinant viral vector. Such a vector is operatively linked to a recombinant nucleic acid sequence encoding a protein and the resulting recombinant nucleic acid molecule is packaged in a viral coat to form a recombinant virus. The protein can be expressed by the recombinant virus in a host cell in an animal *in vivo* and/or *ex vivo* after administration. When administered to an animal, a recombinant virus infects cells at the site of attachment of the connective tissue to bone and directs the production of an attachment-enhancing protein. A number of recombinant viral vectors can be used, including, but not limited to, those based on alphaviruses, poxviruses, adenoviruses, herpesviruses, lentiviruses, adeno-associated viruses and retroviruses. Particularly preferred viral vectors are those based on adenoviruses and adeno-associated viruses. Viral vectors suitable for gene delivery are well known in the art and can be selected by the skilled artisan for use in the present invention. A detailed discussion of current viral vectors is provided in "Molecular Biotechnology," Second Edition, by Glick and Pasternak, ASM Press, Washington D.C., 1998, pp. 555-590, the entirety of which is incorporated herein by reference.

An adenoviral vector infects a wide range of nondividing human cells and has been used extensively in live vaccines without adverse side effects. Adenoviral vectors do not integrate into the host genome, and therefore, gene therapy using this system requires periodic administration, although methods have been described which extend the expression time of adenoviral transferred genes, such as administration of antibodies directed against T cell receptors at the site of expression (Sawchuk et al., 1996, Hum. Gene. Ther. 7:499-506). For use in the present invention, long term expression may not be desirable, since after a connective tissue-to-bone attachment is completely formed, continued expression of the attachment-enhancing proteins may not be necessary. Therefore, adenoviral vectors are especially suited to the present method. The efficiency of adenovirus-mediated gene delivery can be enhanced by developing a virus that preferentially infects a particular target cell. For example, a gene for the attachment fibers of adenovirus can be engineered to include a DNA element that encodes a protein domain that binds to a cell-specific receptor. Examples of successful *in vivo* delivery and expression of genes by adenoviral vectors has been demonstrated and is discussed in more detail below.

Yet another type ofviral vector is based on adeno-associated viruses, which are small, nonpathogenic, single-stranded human viruses. This virus can integrate into a specific site on chromosome 19. This virus can carry a cloned insert of about 4.5 kb, and has typically been successfully used to express proteins *in vivo* from 70 days to at least 5 months. Demonstrating that the art is quickly advancing in the area of gene therapy, however, a recent publication by Bennett et al. reported efficient and stable transgene expression by adeno-associated viral vector transfer *in vivo* for greater than 1 year (Bennett et al., 1999, Proc. Natl. Acad. Sci. USA 96:9920-9925).

As discussed above, a recombinant molecule includes an isolated nucleic acid sequence operatively linked to a transcription control sequence. Transcription control sequences are sequences which control the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the host cells useful in the product and method of the present invention. A variety of such transcription control sequences are known to those skilled in the art. Preferred transcription control sequences include those which function in mammalian, bacterial, insect cells, and preferably in mammalian cells. A recombinant nucleic acid molecule can be selectively (i.e., preferentially, substantially exclusively) expressed in a target cell by selecting a transcription control sequence, and preferably, a promoter, which is selectively induced in the target cell and remains substantially inactive in non-target cells.

One or more recombinant nucleic acid molecules encoding an attachment-enhancing protein can be used to produce the protein. In one embodiment, the protein is produced by expressing a recombinant nucleic acid molecule under conditions effective to produce the protein. A preferred method to produce an encoded protein is by transfecting a host cell with one or more recombinant molecules to form a recombinant cell. Suitable host cells to transfect include any mammalian cell that can be transfected. Host cells can be either untransfected cells or cells that are already transfected with at least one nucleic acid molecule. Host cells useful in the present invention can be any cell capable of producing an attachment-enhancing protein. In a preferred embodiment, the host cell itself is useful in enhancing the attachment of connective tissue to bone. A particularly preferred host cell includes a fibrochondrocyte, a chondrocyte, a mesenchymal precursor cell, an osteoblast, a fibroblast, a tendon cell, a ligament cell or any other cell that can serve as a precursor of chondrocytes, osteoblasts, tendon cells or ligament cells.

As described in detail below, a composition for the provision of attachment-enhancing proteins is associated with an attachment matrix, such that the attachment matrix serves, in one capacity, as a delivery vehicle for the composition to be delivered to the site of attachment of connective tissue to bone. Suitable methods for associating a composition containing attachment-enhancing proteins and/or recombinant nucleic acid molecules encoding such proteins with an attachment matrix include any method which allows the proteins and/or recombinant nucleic acid molecules to be delivered to a site of attachment together with an attachment matrix such that the attachment product is effective to enhance the repair and/or regenerate of the attachment of connective tissue to bone at the site, including both *ex vivo* and *in vivo* methods. Such methods of association include, but are not limited to, suspension of the composition within the attachment matrix, freeze-drying of the composition onto a surface of the matrix and suspension within the matrix of a carrier/delivery formulation containing the composition. Additionally, the composition can be associated with the matrix prior to placement of the product at a site of attachment (i.e., the association of the composition with matrix occurs *ex vivo*) or alternatively, an attachment matrix can first be implanted into the *in vivo* site, followed by association of the composition with the matrix, such as by injection into or on top of the matrix (i.e., the association of the composition with matrix occurs *in vivo*). A composition can contain additional delivery formulations or carriers which enhance the association of the composition with the matrix, which enhance the delivery of the composition to the appropriate cells and tissue at the site of the lesion, and which assist in controlling the release of the factors in the composition at the site ofthe lesion. Suitable delivery formulations include delivery vehicles, which, as used herein, include compounds that increase the half-life of a composition in the treated animal. Suitable delivery vehicles are described in detail below.

According to the present invention, *"ex vivo"* refers to performing part of the regulatory step outside of the patient, such as by associating a composition comprising attachment-enhancing proteins with a matrix and then transplanting the matrix into the patient. Alternatively, or in addition, an *ex vivo* method of providing the attachment-enhancing proteins to the patient is by transfecting a population of cells (e.g., cells removed from a patient) with a recombinant molecule comprising a nucleic acid sequence encoding attachment-enhancing proteins under conditions such that the recombinant molecule is subsequently expressed by the transfected cell. The transfected cell is then associated with the attachment matrix, and the transfected cells are returned to the patient. Methods to achieve such transfection include, but are not limited to, transfection, viral infection, electroporation, lipofection, bacterial transfer, spheroplast fusion, and adsorption.

Alternatively, an attachment matrix can be transplanted at the site of attachment of in the patient, and the composition can be administered such that the proteins and/or recombinant nucleic acid molecules are delivered to and associate with the matrix *in vivo.* Methods of *in vivo* administration include, but are not limited to, intravenous administration, intraperitoneal administration, intramuscular administration, subcutaneous administration, transdermal delivery, intraarticular administration, inhalation (e.g., aerosol), oral, impregnation of a catheter, and direct injection into a tissue. Preferred methods of *in vivo* administration include intraarticular injection, impregnation using a catheter, and direct injection into the implanted matrix.

According to the method of the present invention, when the attachment-enhancing proteins are provided as a recombinant nucleic acid molecule, a host cell is preferably transfected *in vivo* (i.e., in a mammal) as a result of administration to a mammal of a recombinant nucleic acid molecule, or *ex vivo,* by removing cells from a mammal and transfecting the cells with a recombinant nucleic acid molecule *ex vivo.* Transfection of a nucleic acid molecule into a host cell according to the present invention can be accomplished by any method by which a nucleic acid molecule can be administered into the cell *in vivo* or *ex vivo,* and includes, but is not limited to, transfection, electroporation, microinjection, lipofection, adsorption, and viral infection. The resulting recombinant host cell can then be associated with the attachment matrix of the present invention, if it is not already associated with such matrix, by any suitable method to provide (in this case, express) the attachment-enhancing proteins.

In order to transfect a recombinant nucleic acid molecule into a host cell, the recombinant molecule is delivered to the target host cell. As discussed above, recombinant nucleic acid molecules can be delivered to a host cell either *ex vivo* whereby the host cell is then associated with the attachment matrix, or *in vivo* whereby the host cell is a cell that is in the local environment of the attachment matrix. Suitable *in vivo* and/or *ex vivo* delivery methods for a recombinant nucleic acid molecule ofthe present invention include, but are not limited to: (a) administering a naked (i.e., not packaged in a viral coat or cellular membrane) nucleic acid molecule (e.g., as naked DNA or RNA molecules, such as is taught, for example in Wolff et al., 1990, Science 247, 1465-1468); (b) administering a nucleic acid molecule packaged as a recombinant virus or a recombinant cell (i.e., the nucleic acid molecule is delivered by a viral or cellular vehicle), whereby the virus or cell is associated with the attachment matrix; or (c) administering a recombinant nucleic acid molecule associated with the attachment matrix via a delivery vehicle such as a liposome or nanosphere delivery system described herein. Other suitable delivery vehicles useful in the present invention include gold particles, poly-L-lysine/DNA-molecular conjugates, and artificial chromosomes. Certain of the above-described delivery vehicles can also be used to delivery a protein of the present invention to an attachment matrix and/or associate a protein with the attachment matrix. Such delivery vehicles include, for example, a liposome and a nanosphere delivery vehicle.

Delivery of recombinant molecules in a non-targeting carrier (e.g., as "naked" DNA molecules, is taught, for example in Wolff et al., 1990, Science 247, 1465-1468). Such recombinant nucleic acid molecules are typically injected by direct or intramuscular administration. Recombinant nucleic acid molecules to be administered by naked DNA administration include a nucleic acid molecule encoding an attachment-enhancing protein according to the present invention, and preferably include a recombinant molecule of the present invention that is replication, or otherwise amplification, competent. A naked nucleic acid reagent of the present invention can comprise one or more nucleic acid molecule in the form of, for example, a dicistronic recombinant molecule. Naked nucleic acid delivery can include intramuscular, subcutaneous, intradermal, transdermal, intranasal and oral routes of administration, with direct injection into the target tissue being most preferred. A preferred single dose of a naked nucleic acid vaccine ranges from about 1 nanogram (ng) to about 100 µg, depending on the route of administration and/or method of delivery, as can be determined by those skilled in the art. In one embodiment, pure DNA constructs cover the surface of gold particles (1 to 3 µm in diameter) and are propelled into skin cells or muscle with a "gene gun." Several publications by Dzau and collaborators demonstrate the successful *in vivo* delivery and expression of a gene into cells of the heart, including cardiac myocytes, fibroblasts and vascular smooth muscle cells using naked DNA or Hemagglutinating virus of Japan-liposome delivery, administered by both incubation within the pericardium and infusion into a coronary artery (intracoronary delivery) (See, for example, Aoki et al., 1997, J. Mol. Cell, Cardiol. 29:949-959; Kaneda et al., 1997, Ann N. Y. Acad. Sci. 811:299-308; and von der Leyen et al., 1995, Proc Natl Acad Sci USA 92:1137-1141).

Delivery of numerous nucleic acid sequences has been accomplished by administration of viral vectors encoding the nucleic acid sequences. Using such vectors, successful delivery and expression has been achieved using *ex vivo* delivery (See, of many examples, retroviral vector; Blaese et al.,1995, Science 270:475-480; Bordignon et al.,1995, Science 270:470-475), nasal administration (CFTR-adenovirus-associated vector), intracoronary administration (adenoviral vector and Hemagglutinating virus of Japan, see Dzau and collaborators above), intravenous administration (adeno-associated viral vector; Koeberl et al., 1997, Proc Natl Acad Sci USA 94:1426-1431). Gene delivery to synovial lining cells and articular joints has had documented success. Oligino and colleagues report the use of a herpes simplex viral vector which is deficient for the immediate early genes, ICP4, 22 and 27, to deliver and express two different receptors in synovial lining cells *in vivo* (Oligino et al., 1999, Gene Ther. 6:1713-1720). The herpes vectors were administered by intraarticular injection. Kuboki et al. used adenoviral vector-mediated gene transfer and intraarticular injection to successfully and specifically express a gene in the temporomandibular joints of guinea pigs *in vivo* (Kuboki et al., 1999, Arch. Oral. Biol. 44:701-709). Apparailly and colleagues systemically administered adenoviral vectors encoding IL-10 to mice and demonstrated successful expression of the gene product and profound therapeutic effects in the treatment of experimentally induced arthritis (Apparailly et al., 1998, J. Immunol. 160:5213-5220). In another study, murine leukemia virus-based retroviral vector was used to deliver (by intraarticular injection) and express a human growth hormone gene both *ex vivo* and *in vivo* (Ghivizzani et al.,1997, Gene Ther. 4:977-982). This study showed that expression by *in vivo* gene transfer was at least equivalent to that of the *ex vivo* gene transfer. As discussed above, Sawchuk et al. has reported successful *in vivo* adenoviral vector delivery of a gene by intraarticular injection, and prolonged expression of the gene in the synovium by pretreatment of the joint with anti-T cell receptor monoclonal antibody (Sawchuk et al., 1996, *ibid.* Finally, it is noted that *ex vivo* gene transfer of human interleukin-1 receptor antagonist using a retrovirus has produced high level intraarticular expression and therapeutic efficacy in treatment of arthritis, and is now entering FDA approved human gene therapy trials (Evans and Robbins, 1996, Curr. Opin. Rheumatol. 8:230-234). Therefore, the state of the art in gene therapy has led the FDA to consider human gene therapy an appropriate strategy for the treatment of at least arthritis. Taken together, all of the above studies indicate that delivery and expression of protein encoded by a recombinant nucleic acid molecule according to the present invention is feasible.

Another mode of delivery of a recombinant nucleic acid molecule or in one embodiment to a desired target site or cell is by liposome delivery. In this embodiment, a recombinant nucleic acid molecule of the present invention is administered to a patient in a liposome delivery vehicle, whereby the recombinant nucleic acid molecule enters the host cell (i.e., the target cell) by lipofection. According to the present invention, a liposome delivery vehicle comprises a lipid composition that is capable of delivering a recombinant nucleic acid molecule of the present invention, including both plasmids and viral vectors, to a suitable cell and/or tissue in a patient. A liposome delivery vehicle ofthe present invention comprises a lipid composition that is capable of fusing with the plasma membrane of the target cell to deliver the recombinant nucleic acid molecule into a cell.

A liposome delivery vehicle of the present invention can be modified to target a particular site in a mammal (i.e., a targeting liposome), thereby targeting and making use of a nucleic acid molecule of the present invention at that site. Suitable modifications include manipulating the chemical formula of the lipid portion of the delivery vehicle. Manipulating the chemical formula of the lipid portion of the delivery vehicle can elicit the extracellular or intracellular targeting of the delivery vehicle. For example, a chemical can be added to the lipid formula of a liposome that alters the charge of the lipid bilayer of the liposome so that the liposome fuses with particular cells having particular charge characteristics. Other targeting mechanisms include targeting a site by addition of exogenous targeting molecules (i.e., targeting agents, including, but not limited to, antibodies, soluble receptors or ligands), incorporated with the liposome, to target a particular cell or tissue to which the targeting molecule can bind. Targeting liposomes are described, for example, in Ho et al., 1986, Biochemistry 25: 5500-6; Ho et al., 1987a, J Biol Chem 262: 13979-84; Ho et al., 1987b, J Biol Chem 262: 13973-8; and U.S. Patent No. 4,957,735 to Huang et al., each of which is incorporated herein by reference in its entirety).

A liposome delivery vehicle is preferably capable of remaining stable in a patient for a sufficient amount of time to deliver a nucleic acid molecule of the present invention to a preferred site in the patient (i.e., a target cell). A liposome delivery vehicle of the present invention is preferably stable in the patient into which it has been administered for at least about 30 minutes, more preferably for at least about 1 hour and even more preferably for at least about 24 hours. A preferred liposome delivery vehicle of the present invention is from about 0.01 microns to about 1 microns in size.

Suitable liposomes for use with the present invention include any liposome. Preferred liposomes of the present invention include those liposomes commonly used in, for example, gene delivery methods known to those of skill in the art. Preferred liposome delivery vehicles comprise multilamellar vesicle (MLV) lipids and extruded lipids. Methods for preparation of MLV's are well known in the art and are described, for example, in the Examples section. According to the present invention, "extruded lipids" are lipids which are prepared similarly to MLV lipids, but which are subsequently extruded through filters of decreasing size, as described in Templeton et al., 1997, Nature Biotech., 15:647-652, which is incorporated herein by reference in its entirety. Small unilamellar vesicle (SUV) lipids can also be used in the composition and method of the present invention. In one embodiment, liposome delivery vehicles comprise liposomes having a polycationic lipid composition (i.e., cationic liposomes) and/or liposomes having a cholesterol backbone conjugated to polyethylene glycol. In a preferred embodiment, liposome delivery vehicles useful in the present invention comprise one or more lipids selected from the group of DOTMA, DOTAP, DOTIM, DDAB, and cholesterol.

Preferably, the transfection efficiency of a nucleic acid:liposome complex of the present invention is at least about 1 picogram (pg) of protein expressed per milligram (mg) of total tissue protein per microgram (µg) of nucleic acid delivered. More preferably, the transfection efficiency of a nucleic acid:liposome complex of the present invention is at least about 10 pg of protein expressed per mg of total tissue protein per µg of nucleic acid delivered; and even more preferably, at least about 50 pg of protein expressed per mg of total tissue protein per µg of nucleic acid delivered; and most preferably, at least about 100 pg of protein expressed per mg of total tissue protein per µg of nucleic acid delivered.

Complexing a liposome with a nucleic acid molecule of the present invention can be achieved using methods standard in the art. A suitable concentration of a nucleic acid molecule of the present invention to add to a liposome includes a concentration effective for delivering a sufficient amount of recombinant nucleic acid molecule into a target cell of a patient such that the attachment-enhancing protein encoded by the nucleic acid molecule can be expressed in an amount effective to contribute to the enhancement of attachment of connective tissue to bone in a patient. Preferably, from about 0.1 µg to about 10 µg of nucleic acid molecule of the present invention is combined with about 8 nmol liposomes. In one embodiment, the ratio of nucleic acids to lipids (µg nucleic acid:nmol lipids) in a composition of the present invention is preferably at least from about 1:10 to about 6:1 nucleic acid:lipid by weight (i.e., 1:10= 1 µg nucleic acid:10 nmol lipid).

Using liposome delivery, U.S. Patent No. 5,705,151, issued January 6,1998, to Dow et al. demonstrated the successful *in vivo* intravenous delivery of a nucleic acid molecule encoding a superantigen and a nucleic acid molecule encoding a cytokine in a cationic liposome delivery vehicle, whereby the encoded proteins were expressed in tissues of the animal, and particularly in pulmonary tissues. As discussed above, Liu et al., 1997, *ibid.* demonstrated that intravenous delivery of cholesterol-containing cationic liposomes containing genes preferentially targets pulmonary tissues and effectively mediates transfer and expression of the genes *in vivo.* Moreover, as discussed above, Dzau and collaborators have demonstrated the successful *in vivo* delivery and expression of a gene into cells of the heart using Hemagglutinating virus of Japan-liposome delivery, administered by both incubation within the pericardium and infusion into a coronary artery.

Yet another method of delivering either a recombinant nucleic acid molecule encoding an attachment-enhancing protein or an isolated attachment-enhancing protein to a host cell is by using a nanosphere delivery vehicle. A nanosphere delivery vehicle according to the present invention includes the nanosphere delivery vehicle described in European Patent Application No. 0 896 825 A1, published February 17, 1999, which is incorporated herein by reference in its entirety. In a preferred embodiment, such a delivery vehicle includes polymer particles having a size of less than 1000 nm and being loaded with between 0.001% and 17% by weight ofthe attachment-enhancing composition. The nanospheres have an *in vitro* analytically determined release rate profile with an initial burst of about 10% to about 20% of the total amount of the composition over a first 24 hour period, and a long time release rate of a least 0.1% per day during at least seven following days. A nanosphere delivery vehicle is considered to be one type of a controlled release vehicle that is capable of slowly releasing a composition (proteins and/or recombinant nucleic acid molecules) of the present invention into an animal. As used herein, a controlled release formulation comprises recombinant nucleic acid molecule of the present invention in a controlled release vehicle. Other suitable controlled release vehicles include, but are not limited to, biocompatible polymers, other polymeric matrices, capsules, microcapsules, microparticles, bolus preparations, osmotic pumps, diffusion devices, liposomes, lipospheres, and transdermal delivery systems. Controlled release formulations of the present invention can include liquids that, upon association with the matrix or upon administration to an animal, form a solid or a gel *in situ.* Such controlled release vehicles are preferably associated with the attachment matrix by one of the above-described methods. Preferred controlled release formulations are biodegradable (i.e., bioerodible).

A preferred controlled release formulation of the present invention is capable of releasing a composition of the present invention at the site of attachment of connective tissue to bone at a constant rate sufficient to attain therapeutic dose levels of the attachment-enhancing proteins provided by the composition to result in enhancement of the attachment of connective tissue to bone. A particularly preferred controlled release vehicle according to the present invention is a nanosphere delivery vehicle described above.

In addition to the delivery vehicles described above, a composition of the present invention can also include a pharmaceutically acceptable excipient. Suitable excipients of the present invention include excipients or formularies that transport or help transport, but do not specifically target a nucleic acid molecule to a cell (also referred to herein as non-targeting carriers). Examples of pharmaceutically acceptable excipients include, but are not limited to water, phosphate buffered saline, Ringer's solution, dextrose solution, serum-containing solutions, Hank's solution, other aqueous physiologically balanced solutions, oils, esters and glycols. Aqueous carriers can contain suitable auxiliary substances required to approximate the physiological conditions of the recipient, for example, by enhancing chemical stability and isotonicity.

Suitable auxiliary substances include, for example, sodium acetate, sodium chloride, sodium lactate, potassium chloride, calcium chloride, and other substances used to produce phosphate buffer, Tris buffer, and bicarbonate buffer. Auxiliary substances can also include preservatives, such as thimerosal, ― or o-cresol, formalin and benzol alcohol. Compositions of the present invention can be sterilized by conventional methods and/or lyophilized.

A composition is present in the attachment product of the present invention at a concentration that is effective to induce, at the site of attachment of connective tissue to bone, one or more of: complex fan morphology and transition zones of fibrocartilage and calcified cartilage between the connective tissue and bone, induction of cellular infiltration into the product, induction of cellular proliferation, and production of cellular and spatial organization to form a three-dimensional tissue that more nearly represents endogenous connective tissue to bone attachment. Preferably, a composition is present in the attachment product of the present invention at a concentration that is effective to induce the formation of a bone-cartilage-connective tissue interface at the site of attachment. When the attachment-enhancing proteins are provided by the composition directly as a protein, the composition is typically provided at a concentration of from about 0.5% to about 33% by weight of the attachment product. More preferably, the composition is provided at a concentration of from about 1% to about 20% by weight of the attachment product. When one or more of the attachment-enhancing proteins are provided by the composition as a recombinant nucleic acid molecule, an appropriate concentration of a nucleic acid molecule expressing one attachment-enhancing protein is an amount which results in at least about 1 pg of protein expressed per mg of total tissue protein at the site of delivery per µg of nucleic acid delivered, and more preferably, an amount which results in at least about 10 pg of protein expressed per mg of total tissue protein per µg of nucleic acid delivered; and even more preferably, at least about 50 pg of protein expressed per mg of total tissue protein per µg of nucleic acid delivered; and most preferably, at least about 100 pg of protein expressed per mg of total tissue protein per µg of nucleic acid delivered. One of skill in the art will be able to adjust the concentration of proteins and/or nucleic acid molecules in the composition depending on the types and number of proteins to be provided by the composition, and the delivery vehicle used.

In another embodiment ofthe product ofthe present invention, a composition can also contain a factor that non-covalently attaches to one or more of any of the attachment-enhancing proteins or recombinant nucleic acid molecules in the composition and thus, modify the release rate of the factor. Such factors include, but are not limited to, any ground substance or a synthetic polymeric substance. As used herein, a ground substance is defined as the non-living matrix of connective tissue, which includes natural polymers and proteoglycans. Accordingly, a ground substance is a naturally occurring substance, although such substance can be produced synthetically, once the formula and structure is known. Natural polymers include, but are not limited to collagen, elastin, reticulin and analogs thereof. Proteoglycans include, but are not limited to any glycosaminoglycan-containing molecules, and include chondroitin sulfate, dermatan sulphate, heparan sulphate, keratan sulphate and hyaluronan. Preferred ground substances include, but are not limited to, type I collagen, type II collagen, type III collagen, type IV collagen and hyaluronic acid. Preferred synthetic polymeric substances include, but are not limited to, poly(lactic acid) and poly(glycolic acid). In a preferred embodiment, the factor is a glycosaminoglycan.

In a further embodiment, the composition can include one or more types of cells which are provided to further enhance the attachment of connective tissue to bone at the site of the attachment. Such cells include, but are not limited to, fibrochondrocyte, a chondrocyte, a mesenchymal precursor cell, an osteoblast, a fibroblast, a tendon cell, a ligament cell or any other cell that can serve as a precursor of chondrocytes, osteoblasts, tendon cells or ligament cells. Such cells can be associated with the composition and the matrix by any of the methods described above. In one embodiment, at least some of the cells are transformed with a recombinant nucleic acid molecule encoding an attachment-enhancing protein to form a recombinant cell. In this embodiment, the recombinant cell is associated with the attachment matrix *ex vivo* or *in vivo* by any suitable method as described previously herein. In addition, or alternatively, the product of the present invention can include cells that have been cultured with a mixture of the attachment-enhancing proteins as previously described herein.

Preferably, the cells to be cultured with the mixture of proteins are cells which are involved in the formation of a bone-cartilage-connective tissue interface at the site of attachment of connective tissue (i.e., tendon and/or ligament) to bone, and include, but are not limited to, fibrochondrocytes, chondrocytes, mesenchymal precursors, osteoblasts, fibroblasts, and any other cell that can serve as a chondrocyte precursor. Such cells are preferably cultured *in vitro* (i.e., *ex vivo*) prior to their association with an attachment matrix, under conditions effective to allow the cells to interact with the proteins and initiate differentiation in the cells. Effective culture conditions include, but are not limited to, effective media, bioreactor, temperature, pH and oxygen conditions that permit interaction of the proteins and cells and initiation of differentiation and proliferation processes by the cells. An effective, medium refers to any medium in which a cell is cultured provide such a result. Such medium typically comprises an aqueous medium having assimilable carbon, nitrogen and phosphate sources, and appropriate salts, minerals, metals and other nutrients, such as vitamins. Cells can be cultured in conventional fermentation bioreactors, shake flasks, test tubes, microtiter dishes, and petri plates. Culturing can be carried out at a temperature, pH and oxygen content appropriate for a the cell. Such culturing conditions are within the expertise of one of ordinary skill in the art. In another aspect of this embodiment of the present invention, an attachment matrix is cultured *in vitro* (i.e., *ex vivo*) together with the cells and mixture of proteins prior to implantation at a site of attachment of connective tissue to bone *in vivo.* In a further embodiment, the cells that have been cultured with the mixture of proteins can be associated with the attachment matrix in conjunction with additional attachment-enhancing proteins and/or recombinant nucleic acid molecules encoding such proteins as described above.

The product for enhancing the attachment of connective tissue to bone of the present invention also includes an attachment matrix configured to interface between connective tissue and bone. The attachment matrix is the component of the product which provides a vehicle for delivery of the composition of the present invention to the site of attachment of connective tissue to bone, and the matrix also provides a suitable scaffold upon which the attachment of connective tissue to bone and the formation of a substantially physiologically normal bone-cartilage-connective tissue interface can form. In a preferred embodiment, the attachment matrix is bioresorbable.

According to the present invention, a matrix which is "configured to interface between connective tissue and bone" is a matrix which, either prior to or at the time of implanting the matrix into the patient, is of a shape or manipulated into a shape, which is suitable for the purpose of attaching a tendon or ligament to bone. For example, in one embodiment, the matrix is in the form of a sheet, which is configured to interface between connective tissue and bone by wrapping the sheet around the tendon or ligament base in such a manner that the sheet can form an interface with bone at the attachment site on the bone. In another embodiment, the matrix is configured to have the dimensions of a tendon or ligament base that is in contact with a bone trough. In another embodiment, the matrix is a gel that is injected around a tendon in tendon tunnels and between tendon and bone, thereby conforming to the attachment site as the matrix is applied to the site of attachment. In this embodiment, preferably, the gel is buffered so that minimal cell or tissue necrosis occurs when the product is implanted into the body.

According to the present invention, an attachment matrix can be formed of any material that is suitable for *in vivo* use, and which provides the above-described characteristics of an attachment matrix for use with a composition of the present invention. The matrix can be formed of materials which include, but are not limited to, synthetic polymeric material and/or a ground substance (defined previously herein). Preferred ground substances include natural polymers and proteoglycans. Natural polymers include, but are not limited to collagen, elastin, reticulin and homologues thereof. Proteoglycans include, but are not limited to, any glycosaminoglycan-containing molecules. Particularly preferred glycosaminoglycans include chondroitin sulfate, dermatan sulphate, heparan sulphate, keratan sulphate and hyaluronan. Other preferred ground substances include, but are not limited to, type I collagen, type II collagen, type III collagen, type IV collagen and hyaluronic acid. Preferred synthetic polymers include poly(lactic acid) and poly(glycolic acid).

In one embodiment ofthe present invention, the attachment matrix includes collagen. Preferably, the matrix contains from about 20% to about 100% collagen by dry weight of the matrix, and more preferably, from about 50% to about 100% collagen by dry weight of the matrix, and even more preferably, from about 75% to about 100% collagen by dry weight of the matrix. In one embodiment, a suitable attachment matrix includes collagen from bovine tendon.

An attachment matrix suitable for use in the present invention can include a material as described above which is in any suitable form for use in attaching connective tissue to bone, including a sponge, a membrane, a film or a gel. In one embodiment, a suitable attachment matrix includes autograft tissue, allograft tissue and/or xenograft tissue.

An attachment product of the present invention is useful for repairing or regenerating connective tissue-to-bone attachments where the attachment has been completely orpartially severed as a result of an injury or surgical procedure, or has deteriorated due to a degenerative condition or disease, for example. The product is particularly useful for attaching to a bone structure a ligament selected from the group of an anterior cruciate ligament, an ulnar collateral ligament or a lateral ankle ligament. The product is also particularly useful for attaching to a bone structure a tendon selected from the group of supraspinatus tendon, infraspinatus tendon, subscapularis tendon, and teres minor tendon.

Various defects in tendons and ligaments have been well defined in the art. For example, Harryman et al. defined the status of the rotator cuff with respect to the tendon integrity (Harryman et al., 1991, "Repairs of the Rotator Cuff", J. Bone Joint Surgery 982-989). Type 0 refers to an intact cuff, type 1B refers to a full-thickness defect of the supraspinatus tendon, type 2, to a full-thickness defect involving the supraspinatus and infraspinatus tendons, and type 3 to a full-thickness defect involving the supraspinatus, infraspinatus and subscapularis tendons. Tendon etiology also involves partial thickness tears of these tendons.

Therefore, since defects or injuries can occur in a variety of shapes, sizes, and locations, an attachment matrix suitable for use in an attachment product of the present invention is of a shape and size sufficient to conform to a specific attachment of connective tissue to bone in the patient to be treated. Preferably, the attachment matrix, when used in the attachment of connective tissue to bone, achieves a geometry (i.e., has dimensions) that is suitable to provide a therapeutic benefit to the patient. Such a therapeutic benefit can be any improvement in a patient's health and well being that is related to a correction of defect in the attachment, and preferably, the therapeutic benefit includes the reattachment of the connective tissue to bone such that the natural configuration of the attachment is at least partially restored.

As discussed above, in one embodiment, the attachment matrix has dimensions which correspond to a tendon or ligament base that is in contact with a bone trough. In another embodiment, the attachment matrix is configured as a sheet. The sheet is preferably of a shape and size suitable for wrapping around a tendon or ligament to be reattached to a bone. Preferably, the matrix provides or enhances an immediate mechanical attachment of the connective tissue to the bone, and also provides a scaffold upon which formation of a connective tissue-fibrocartilage/calcified cartilage-bone interface can occur. Additionally, the matrix preferably imparts upon the product a mechanical stability sufficient to allow the product to be anchored at the site of attachment. The matrix can be non-crosslinked or crosslinked with chemical and/or physical crosslinking methods known to those skilled in the art. Sufficient crosslinking is performed so that it confers mechanical stability, but does not substantially impede cellular infiltration. In one embodiment of the present invention, when the product is used to attach the anterior cruciate ligament (ACL) to the femoral and tibial tunnels, the attachment matrix is preferably configured as a sheet, and preferably has the dimensions to completely wrap around the ACL. In another embodiment of the present invention, when the product is used to repair a rotator cuff injury, the attachment matrix preferably has the corresponding dimensions of the tendon base that is in contact with the bone trough of the humerus.

In one embodiment, the attachment matrix has a thickness of from about 0.1 mm to about 3 mm, and more preferably, from about 0.5 mm to about 2 mm. The thickness can, of course, be varied depending on the configuration of the connective tissue and bone attachment site. In this embodiment, the matrix can be prepared by applying an aqueous dispersion of matrix material into a mold, for example, wherein the mold sets the appropriate thickness for the sheet. Such a method is described in Example 1. In a preferred embodiment, the matrix is prepared from an aqueous dispersion of from about 0.2% to about 4% collagen by weight, and more preferably, the matrix is prepared from an aqueous dispersion of from about 0.5% to about 3% collagen by weight. To obtain a 2% collagen by weight sponge, a 2% collagen dispersion is made and lyophilized.

In a preferred embodiment, the attachment matrix of the present invention is porous, which enhances the ability of the matrix to serve as a delivery vehicle for the attachment-enhancing composition and particularly, as a scaffold upon which cellular processes leading to formation of a connective tissue-fibrocartilage/calcified cartilage-bone interface can occur, such as by allowing for the ingrowth of cells into the matrix. Preferably, the pore size is sufficient to maintain the desired mechanical strength ofthe matrix, while allowing sufficient ingrowth of cells for regeneration of the desired interface at the site of attachment. The porosity of the matrix can vary depending on the configuration of the matrix, but typically, the matrix has a pore size of from about 10 µm to about 500 µm. In one embodiment, and particularly when the matrix is configured as a sheet, the matrix has a preferred pore size of from about 10 µm to about 100 µm.

One embodiment of the present invention relates to a method of using the product of the present invention for enhancing an attachment of connective tissue to bone. This method includes the steps of implanting and fixing at a site of attachment of connective tissue to bone, a product comprising: (a) an attachment matrix; and, (b) a composition associated with the attachment matrix for provision of attachment-enhancingproteins. Suitable compositions for use in a product of the present invention have been described in detail above, and all such compositions can be used in the present method. The use of the product of the present invention in the method for enhancing attachment is effective to induce the formation of a bone-cartilage-connective tissue interface at the site of attachment of connective tissue to bone. Also as discussed above, as used herein, the term "connective tissue" refers to connective tissue selected from the group of tendon and ligament.

The method of the present invention is useful for attaching any connective tissue selected from the group of tendon and ligament to bone, including attaching the anterior cruciate ligament to the femoral and tibial tunnels, attaching the ulnar collateral ligament to the ulna, or attaching the lateral ankle ligament to the tibia. The method is also particularly useful for attaching a supraspinatus tendon to the greater tuberosity of the humerus, an infraspinatus tendon to the greater tuberosity of the humerus, a subscapularis tendon to the lesser tuberosity of the humerus, or a teres minor tendon to the greater tuberosity of the humerus. The step of implanting is performed using surgical techniques known in the art, and typically involves wrapping the product around the connective tissue to be attached to bone when the matrix is configured as a sheet, and/or involves a more complex process of removing damaged tissue and/or implanting the product which is configured to correspond to a tendon or ligament base that is in contact with a bone trough to fix the product to the muscle and/or bone.

In one embodiment, the method of the present invention is useful for regenerating the attachment of alveolar bone to cementum. In this embodiment, the use of the product of the present invention is effective to induce the formation of a bone-fibrocartilage/calcified cartilage-cementum interface, which mimics the natural ontogeny of this connection and is a preferred ontogeny. This embodiment of the present invention is useful in patients in which degeneration of the alveolar bone and attached cementum has occurred to restore this connective tissue to bone attachment, and/or as a preventative measure to delay or reduce further degeneration of this connective tissue to bone attachment.

Preferably, the use of a product of the present invention in a method of the present invention results in significantly improved biomechanical strength of the connective tissue to bone attachment which has been repaired or regenerated, as compared to such an attachment which is performed in the absence of a product of the present invention (i.e., an attachment formed using conventional means, such as by suture, or with matrices in the absence of a composition of the present invention). Preferably, the use of the method of the present invention results in a biomechanical strength of the connective tissue-to-bone attachment which is at least about 20%, and more preferably at least about 50%, and more preferably at least about 75%, and even more preferably greater than 100% greater than the biomechanical strength of a connective tissue-to-bone attachment performed in the absence of the product of the present invention, or alternatively, in the absence of a composition of the present invention.

By way of example, one surgical approach for ACL repair is a single incision, endoscopic method (DW Jackson and PR Kurzweil, Chapter 8 in Master Techniques in Orthopaedic Surgery, Reconstructive knee surgery, Raven Press, NY,1995). Briefly, in this technique, the graft is harvested and a collagen sponge is sutured to the tibial end of the graft. Then, notchplasty is performed and the femoral tunnel is marked. The tibial tunnel is prepared followed by drilling of the femoral tunnel. Using guides, a thin, hollow collagen cylinder is placed in the femoral tunnel. The dimensions of the cylinder (closed on one end) are similar to the tunnel dimensions such that the cylinder is in contact with the bone and does not substantially extrude into the articular space. The graft is then implanted. Normal tibial and femoral graft fixation methods are utilized.

The step of fixing can include attaching the product to the bone, existing connective tissue, and/or muscle by any suitable means, including previously described surgical techniques for the repair of damaged or severed tendons and ligaments. Such a means for attaching can include, but is not limited to application of bioresorbable sutures, application of interference screw, endobutton, bioresorbable sutures, compression screw, non-resorbable sutures, press-fitting, arrows, nails, and T-fix suture anchor devices.

It is to be noted that the term "a" or "an" entity refers to one or more of that entity; for example, a protein refers to one or more proteins, or to at least one protein. As such, the terms "a" (or "an"), "one or more" and "at least one" can be used interchangeably herein. It is also to be noted that the terms "comprising", "including", and "having" can be used interchangeably.

The following examples are provided for the purposes of illustration and are not intended to limit the scope of the present invention.

### EXAMPLES

### Example 1

The following example shows a characterization of tissue derived *in vivo* by implantation of an attachment product of the present invention which includes Bone Protein (BP) in the rodent subcutaneous assay.

In this experiment, samples were implanted using the rat subcutaneous model (i.e., a modified version of the rat ectopic implant assay described in Sampath and Reddi,1983, Proc. Natl. Acad. Sci. USA 80:6591-6595). Briefly, collagen sponges were prepared by making a 4% bovine Tendon Type I collagen dispersion in 1% (v/v) acetic acid. This dispersion was incubated at ambient temperature for 12 to 24 hours. The dispersion was placed into the holes of a molding sheet and excess collagen was removed. A glass sheet was placed over the molding sheet and this was placed at -70 °C for one hour. The molding sheet was then freeze-dried for greater than 12 hours. The resulting discs were pressed out of the molding sheet, the edges were trimmed and the discs were weighed. To add BP to the collagen discs, the discs were placed in a holding plate and 100 microliters of the BP solution was placed under the disc. After the sponge was thoroughly wetted, the collagen sponges were placed in a humidifier for 30 minutes, then frozen at -70 ° for greater than one hour, and finally were freeze-dried for greater than 12 hours.

Long-Evans rats were anesthetized with a non-lethal dose of sodium phenobarbital. After shaving the ventral region, two small incisions were made just above the pectoralis muscles. Samples containing BP were placed into the pouches of each animal.

Animals were sacrificed by CO₂ asphyxiation after three weeks. At explant, samples were weighed and examined grossly. They were placed in 100% methanol, embedded in glycol methacrylate, sectioned and stained with H&E, Von Kossa and Toluidine Blue.

The grading scale for bone formation used to evaluate this experiment is shown in Table 1:

**TABLE 1**

| | |
|---|---|
| Zero (0): | - No residual implanted sample found. |
| | - Section shows no silver stained deposits or those deposits are associated with acellular events, (e.g., dystrophic mineralization of collagen fibrils). |
| | - Explants generally small, soft and avascular. |
| One (1): | - Focal areas of silver stained mineralized tissues are of cellular origin. This may include mineralized cartilage as well as mineralized osteoid matrix. |
| | - Silver stained areas are randomly located throughout the explant, and typically encompass less than 50% of the explant. |
| | - Generally smaller than original implants. |
| Two (2): | - Silver stained areas are mineralized cartilage or very early woven bone. |
| | - Osteoblasts appear in rows of only about 6 to 10 cells. |
| | - If osteoid is present, it is generally present on less than 10% of the mineralizing tissue in the section. |
| | - Small areas of hematopoietic marrow elements may be visible (generally sinusoids containing red blood cells). |
| Three (3): | - Sheets of active osteoblasts, (e.g., cells are plump and cuboidal or polygonal) generally consisting of 10 or more cells, appear in less than 50% of the active mineralized portion. They are generally not continuous. |
| | - Bone associated with osteoblasts is generally woven, containing some osteocytes. |
| | - Woven bone appears at outer regions of explant and may have breaks of fibrous tissue or mineralized cartilage <10% of surface. |
| | - Some hematopoietic marrow elements may be visible. (Hemopoietic cords and sinusoids containing red blood cells.) |
| Four (4): | - Mineralized tissue at the periphery is generally not woven, but a mature band containing lamellar bone. |
| | - Mature bone is associated with continuous osteoblast surfaces in at least 50% of bony area. |
| | - Osteoid contains active osteoblasts and a visible osteoid matrix. |
| | - Bone marrow as evidenced by granulocytes, hemopoietic cords and sinusoids is common. |
| | - Evidence of osteoclastic resorption (presence of osteoclasts and/or Howship's lacunae). |
| Five (5): | - Solid rim of mature bone with few breaks around outer edge of explant. |
| | - Mature bone contains osteocytes in organized patterns. |
| | - Mature bone contains wide dark staining (in TBO stain) osteoid. |
| | - Osteoid seams are continuous with few breaks; very tick with osteoblasts that may be flattened. |
| | - Bone marrow contains hemopoietic cords packed with cells, granulocytes, sinusoids and adipocytes. |
| | - Trabecular bone in marrow is resorbing and may appear as focal areas with little branching. |
| | - Osteoclastic resorption is occurring on outer edge of mature bone (presence of osteoclasts and/or Howship's lacunae). |
| | - Explant center may contain mature woven bone or be infarcted and largely acellular. |
| | - No evidence of chondrocytes. |

BP containing explants routinely produce a histology score of 2-3. Von Kossa staining reveals an organized woven bone around the explant periphery. Just interior is hematopoietic marrow that consisted of sinusoids and red blood cells. Lining the trabeculae is the presence of 6-10 to more than 10 aligned osteoblasts. More than 50% of the silver stain is of cellular origin (e.g. is not dystrophic). The amount of continuous woven bone ranges between 0 and 50%.

### Example 2

The following example demonstrates that a product of the present invention which includes Bone Protein enhances the rate of healing of an attachment of the long digital extensor tendon to bone in rabbits, as compared to in the absence of the product or in the absence of the composition portion of the product.

For device preparations, bovine tendon type I collagen was added to 10 mM HCl to produce a 2% collagen slurry. The slurry was mixed extensively in coupled syringes. After an overnight incubation at ambient temperature, the mixture was injected into a mold of approximately 1.2-mm thickness. The mold was then placed at -70°C for one hour and then lyophilized overnight. The sponges were then cut to the appropriate dimensions (6 x 10 mm). The resulting sponges could completely encompass the tendon in circumference. Also, the dimensions were such that the length of the collagen spanned the tunnel distance (*e.g.* there was no collagen outside of the tunnel). For the low dose, BP was contained at 1.75% (35 µg) of the sponge dry weight. For the high dose, BP was contained at approximately 7.5% (150 µg) of the sponge dry weight.

To determine whether BP enhances the rate of tendon to bone healing, the following study was performed using twelve skeletally mature New Zealand white rabbits according to a published model (Rodeo et al., 1993, J. Bone Joint Surgery 75-A(12):1795-1803; or Rodeo et al., 1999, Am. J. Sports Med. 27(4):476-488). Briefly, a midline incision was made to expose the knee, a lateral arthrotomy was made, and the extensor mechanism was retracted medially to expose the long digital extensor tendon. The long digital extensor tendon was then detached from the lateral femoral condyle. The tibialis anterior muscle was then detached from the proximal lateral tibia and a 2.0 mm drill hole was made at a 45-degree angle to the long axis of the proximal tibial metaphysis. The long digital extensor tendon was then pulled manually through into the tunnel and sutured to the medial aspect of the proximal tibial metaphysis. Each rabbit received a collagen sponge containing BP in one limb while the contralateral limb received either the collagen sponge only or no implant as controls. The collagen material was wetted, wrapped around, and sutured to the tendon. Two different BP doses were used. Six rabbits received each dose. Both the dose and the limb which received the test agent, was randomized. The animals were allowed free cage activity. The animals were sacrificed at 2 weeks and the tissues were prepared for histological analysis. The specimens were fixed in 10% neutral buffered formalin and embedded in methylmethacrylate without decalcification. Histologic sections were stained with H&E and by the Von Kossa method. The treatment groups are shown in Table 2.

**TABLE 2**

| LIMB ONE | LIMB TWO | # ANIMALS |
|---|---|---|
| 35 µg BP + collagen | collagen | 3 |
| 35 µg BP + collagen | no implant | 3 |
| 150 µg BP + collagen | collagen | 3 |
| 150 µg BP + collagen | no implant | 3 |

Histological analysis demonstrated that the samples containing either high or low BP dose showed newly formed bone in greater proximity to the tendon than either of the controls (data not shown). Furthermore, BP-treated areas contained fibrocartilage in close juxtaposition to the tendon, whereas the controls showed only fibrous tissue juxtaposed to the tendon.

Histomorphometric analysis was also performed. The tendon area, tendon circumference, bone area, bone marrow area, and bone perimeter were measured. There were no statistical differences among high dose, low dose, collagen control, or untreated control for tendon area or tendon circumference. In contrast, for bone marrow area, bone area, and bone perimeter, the low dose was statistically greater than the collagen control using the analysis of variance (p < 0.05), Kruskal-Wallis One-Way Anova, and the Kruskal-Wallis multiple-comparison Z value tests. In addition, both the high and low dose BP treatments showed greater bone marrow area, bone perimeter, and bone area than the untreated control, although the differences were not statistically significant. The low dose BP also scored higher than the high dose BP for these parameters, although these differences were also not statistically significant.

### Example 3

The following example shows that a product of the present invention which includes Bone Protein enhances the rate and quality of healing of graft (semitendinosus tendon) attachment to bone in an anterior cruciate ligament reconstruction surgery, as compared to in the absence of the product or in the absence of the composition portion of the product.

For the device preparation, bovine tendon type I collagen was added to 10 mM HCl to produce a 2% collagen (by weight) slurry. The slurry was mixed extensively in coupled syringes. After an overnight incubation at ambient temperature, the mixture was injected into a mold of approximately 1.2-mm thickness. The mold was then placed at -70°C for one hour and then lyophilized overnight. The sponges were then cut to the appropriate dimensions (7 x 11 mm). The size of the resulting sponge completely encompassed the tendon in circumference. Also, the dimensions were such that the length of the collagen spanned the tunnel distance (*e.g.,* there was no collagen outside of the tunnel). Bone Protein (BP) was added to the sponges at a dose of approximately 2.4% (55 µg) of the sponge dry weight. Sponges were sutured to both the tibial and femoral grafts.

All rabbits were obtained from a licensed USDA dealer and cared for according to the standards of the National Institutes of Health. Seventy-five rabbits were premedicated with atropine (0.05 mg/kg), ketamine (35 mg/kg), and acetylpromazine (0.5 mg/kg) and then intubated. After induction of general anesthesia both hind limbs were shaved, scrubbed with betadine, and aseptically draped. A vertical midline incision was made over the knee and a medial parapatellar arthrotomy performed to provide exposure to the knee joint. The extensor mechanism was dislocated laterally. The ipsilateral semitendinosus tendon was identified at its insertion to the proximal medial tibia and exposed up to its muscle-tendon junction. The tendon was transected at the muscle-tendon junction. This provides approximately 35-40 mm of tendon length for the planned surgical procedure. No adverse effects were observed due to harvest of the semitendinosus tendon.

After graft harvest the anterior cruciate ligament was excised. A drill was used to place a drill tunnel (1.7 mm) in the proximal medial tibia entering into the joint at the ACL attachment. Another drill tunnel (1.7 mm) was made in the lateral femoral condyle, entering the joint at the femoral attachment of the ACL. The BP-containing sponge was sutured to the portion of the tendon that was placed into the bone tunnels in the femur and tibia. The treatments were randomized and the surgeon was blinded to the treatment group. In each animal, one limb received BP and the contralateral limb received either carrier or no BP. A passing suture was placed through on one end of the tendon graft and used to pull the graft into the bone tunnels. The graft was placed under tension and sutured to the periosteum and surrounding soft tissues over the lateral femoral condyle and proximal tibia. The wound was closed in layers, and the animals allowed ad-lib activity post-operatively. The procedure was performed bilaterally. No significant complications were observed. The animals were monitored in the post-operative period by the veterinary staff. Antibiotics (ampicillin 25 mg/kg IM or SQ) were administered for the first 48 hours post-operatively, and analgesics (buprenorphine 0.05-0.075 mg/kg SQ) were administered as needed. Nine animals were used for histologic evaluation and 16 for biomechanical testing at each of three time points (2, 4, 8 weeks). The rabbits were sacrificed by an overdose of intravenous pentobarbital.

### Histologic analysis:

At the sacrifice time, each specimen was grossly examined for any evidence of degenerative joint changes, synovial inflammation, and effusion. The tibiae and femura were harvested and placed in 10% neutral-buffered formalin. The specimens were embedded in polymethylmethacrylate without decalcification. Five micron thick sections were cut perpendicular to the bone tunnels yielding cross sections of the tendon graft-bone interface of both the femoral and tibial tunnels. Sections were stained with H+E, Von Kossa, and Masson's trichrome, then examined with light and polarized light microscopy on an Olympus BH-2 microscope. Healing between the tendon and bone tunnel was assessed by formation of new tissue (fibrovascular, granulation tissue, cartilage, and bone) between tendon and bone. Collagen fiber continuity between tendon and bone was assessed with polarized light microscopy. In order to detect any adverse effects of the BP treatment, the presence of articular cartilage degeneration, synovial hyperplasia, and foreign-body giant cell response were also assessed. Comparisons were made between groups (BP-treated and control limbs), as well as between different time points.

Gross observations demonstrated no adverse effects of the BP treatment on the joint tissue. Occasional heterotopic bone formation was observed at the extra-articular tunnel entrance in both treated and control specimens, with more extensive bone formation in the BP-treated specimens. Ossification was not seen at the intra-articular aperture of the drill tunnel. No meniscal specimens had evidence of ossification.

Histology showed that, in the two week specimens treated with BP, there was extensive formation of new bone trabeculae and cartilage in the tendon-bone interface. There was much more new bone formation than in the contralateral control specimen, and this new bone was in closer apposition to the tendon. Overall, the amount of cartilage in the tendon-bone interface was similar between treated and control limbs. However, there was less variability in healing in the BP-treated limbs than in the control limbs. Additionally, there was no evidence of foreign body giant cell response in the BP-treated limbs. Finally, there was no evidence of adverse effect on the articular cartilage of the tibia or femur in the BP-treated limbs.

In four week specimens, there was progressive maturation of the interface tissue between tendon and bone in both control and treated specimens. There was generally more cartilage in the tendon-bone interfaces in the BP-treated specimens compared to the controls, and the cartilage in the tendon-bone interfaces was more mature in the BP-treated specimens. Incorporation of the tendon with establishment of collagen fiber continuity between tendon and bone was often more advanced in the BP-treated specimen. Proliferation of cells along the edge of the tendon was apparent in some of the control and treated specimens with no obvious difference between groups.

In 8 week specimens, the BP-treated specimens demonstrated more cartilage and new bone formation around the tendon graft than in the controls.

### Biomechanical testing:

The femur-ACL graft-tibia construct was harvested from each limb and frozen at - 80°C until testing. Before testing, the tibiae and femura were dissected free of soft tissue proximal and distal to the femoral and tibial graft attachment sites, respectively. The bones were potted in bonding cement just above the femoral sutures and below the tibial sutures, allowing secured fixation in the testing jig. A specially designed apparatus was used that allowed the specimens to be oriented so that a uniaxial tensile load was applied along the axis of the reconstructed ligament in the sagittal plane. The construct was loaded on a MTS machine at a strain rate of 40 mm/sec. The ultimate load to failure was recorded.

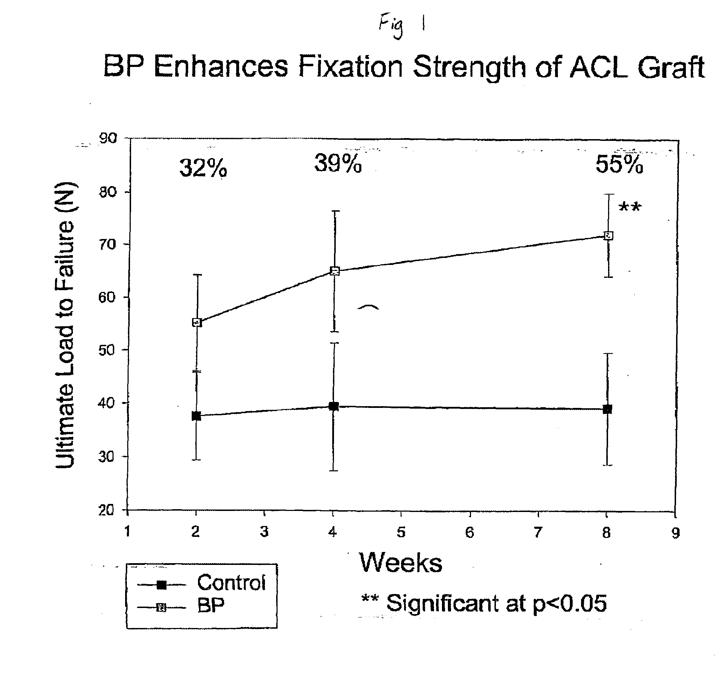

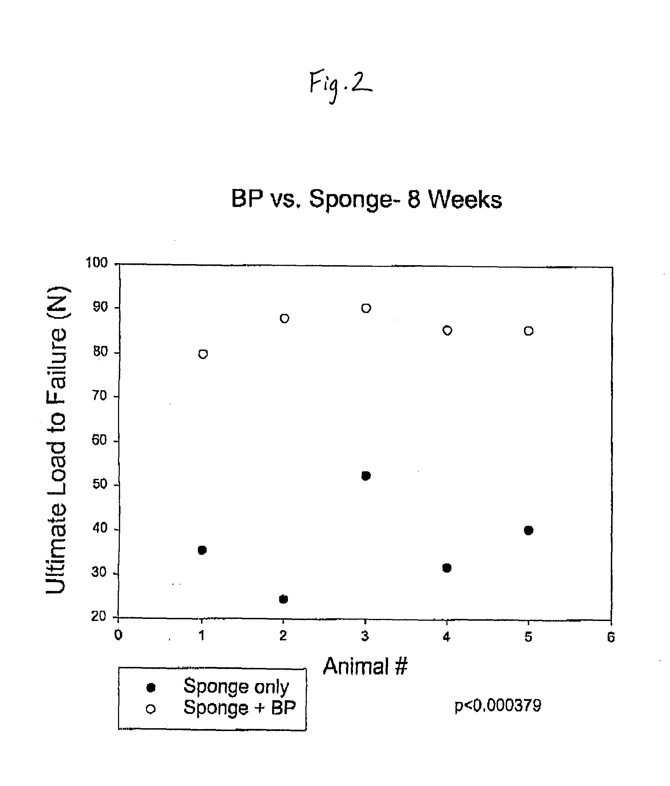

A power analysis revealed that 8 specimens were required at each time point for biomechanical analysis to achieve a power of 0.8 with α=0.05. The ultimate load to failure was compared between the experimental and control limbs using analysis of variance. Comparisons of groups at different time points and between control groups were performed using Student's t-tests. The results are illustrated in Figs. 1-2.

The average load to failure in BP-treated specimens was significantly greater than controls for the entire study population (p<0.001) and at each individual 2, 4, and 8 week time point (p=0.04, 0.01, <0.001, respectively) (See Fig. 1). Also, the BP treated side was significantly stronger (p=0.001) compared with untreated sponge (group I) for the entire population. At individual time points, it is noted that the experimental limb was significantly stronger only at 8 weeks (p=0.0003) (Fig. 2). In addition, the BP treated side was significantly stronger (p=0.02) compared with no sponge (group II), although when compared at each time point, the experimental limb was significantly stronger only at 8 weeks (p=0.05). There were no significant differences between control group I (untreated collagen sponge) and control group II (no sponge).

### Example 4

The following example demonstrates that a product of the present invention which includes Bone Protein enhances the rate of healing of an attachment of infraspinatus tendon to bone in sheep, as compared to in the absence of the product or in the absence of the composition portion of the product.

For the device preparation, bovine tendon type I collagen was added to 10 mM HCl to produce a 2% collagen (by weight) slurry. The slurry was mixed extensively in coupled syringes. After an overnight incubation at ambient temperature, the mixture was injected into a mold of approximately 1.2-mm thickness. The mold was then placed at -70°C for one hour and then lyophilized overnight. The sponges were then cut to the appropriate dimensions (10 x 25 mm). The size of the resulting sponge completely encompassed the base of the infraspinatus tendon. Also, the dimensions were such that no or little collagen was outside of the tendon. The BP dose was approximately 1.7% (170 µg) and 5.7% (570 µg) of the sponge dry weight.

Anesthesia was induced with Ketamine (4 mg/kg) and Valium (7.5 mg total) and maintained on halothane (1.5-3%) in 100% oxygen (2 L/min). Under general anesthesia using aseptic conditions, a 6-cm skin incision was made over the right shoulder joint, the cranial to acromial head of the deltoideus muscle was dissected, and the infraspinatus tendon was detached from the insertion point. A trough, 1.5-cm in length and 0.5 cm depth, was prepared in the proximal humerus using a Hall orthopaedic burr. Four separate drill holes were created in the bone of the humerus surrounding the trough. The infraspinatus tendon was sutured into the trough using #2 non-absorbable suture (Ethibond; Ethicon, Inc.) in a modified Mason-Allen technique. Under the suture, a collagen sponge containing BP was inserted. Alternatively, nothing (no collagen or BP) was placed under the suture (control). The sutures were tied separately over the cortical bridge. Subcutaneous tissues and skin was closed using routine methods. Following surgery, the sheep were monitored until a swallowing reflex was observed and were then transported to the pasture. There, they were propped into sternal recumbency and were monitored throughout the day. At the end of the day, all sheep were returned to the pens. Post-operative analgesics consisted of 1 gram of the non-steriodal, anti-inflammatory drug phenylbutazone (p.o.). In addition, all sheep had two Fentanyl patches (Duragesic, (50Ag/h) Transdermal System, Janssen Pharmaceutic; Titusville, NJ) applied to the lateral thoracic region preoperatively for postoperative pain. In addition to the Fentanyl patches, phenylbutazone (1 gm p.o.) was administered daily for 3 postoperative days.

The animal groups are shown in Table 3.

**TABLE 3**

| ANIMAL | IMPLANT |
|---|---|
| 1 | None |
| 2 | 570 µg BP + collagen |
| 3 | 570 µg BP + collagen |
| 4 | 170 µg BP + collagen |
| 5 | 170 µg BP + collagen |
| 6 | None |

To calculate the percentage of bone or cartilage, the following procedure was performed. Using the histological slide that was stained with Toluidine Blue, the 4X objective was used for viewing. The top line of the photographic rectangular box was then aligned with the bottom edge of the pre-existing bone. The area below the pre-existing bone is new repair tissue. The percentage area of bone and cartilage that filled the box was estimated. The percentage reported in Table 4 is the average of three independent fields. The results demonstrate that BP treated samples induced a higher percentage of newly synthesized bone and cartilage as compared to the control.

**TABLE 4**

| Animal # | % bone or cartilage* | Treatment |
|---|---|---|
| 1 | 33 | Surgical control |
| 6 | 65 | Surgical control |
| 2 | 100 | 570 mcg BP + collagen |
| 3 | 100 | 570 mcg BP + collagen |

| | | |
|---|---|---|
| *% bone or cartilage: average of three areas in 4X magnification. For proper orientation, the top margin of each area should be aligned with pre-existing bone. | | |

### Example 5

The following example demonstrates that bone protein (BP) is a complex mixture of proteins which includes at least: TGFβ1, TGFβ2, TGFβ3, BMP-2, BMP-3, BMP-4, BMP-5, BMP-6, BMP-7, CDMP, FGF-I, osteocalcin, osteonectin, BSP, lysyloxidase, cathepsin L pre, albumin, transferrin, Apo A1 LP and Factor XIIIb.

The present inventors used standard techniques and reagents available in the art to identify these proteins within bone protein (See for example, "Current Protocols in Protein Science", Ed. JE Coligan et al.; 1995-1998, John Wiley and Sons, Inc.; "Protein Purification: Principles and Practice" Scopes and Verlas;1982). The proteins which have been identified as present in BP by at least one of these assays are identified in Table 5.

**TABLE 5**

| BP COMPOSITION | | | | | | |
|---|---|---|---|---|---|---|
| **Compound** | **Mass (kD)** | | **pl** | **Presence in BP** | | |
| | **Literature** | **Experimental** | | **ELISA** | **Immunoblot** | **Mass. Spec./ Sequencing** |
| | | | | | | |
| **TGFβ Superfamily** | | | | | | |
| | | | | | | |
| TGFβ1 | 12.5^{Ob} | 12.5 | | YES | YES | |
| TGFβ2 | 12.7^{T} | 12 | 7.7^{T} | | YES | YES |
| TGFβ3 | | | | | YES | |
| | | | | | | |
| BMP-2 | 16-18^{Ob} | 16 | 7.9^{T} | NO | YES | |
| BMP-3 | 16-18^{Ob} | 31 | 8.5^{T} | YES | YES | |
| BMP-4 | 16-18^{Ob} | | 7.7^{T} | | YES | |
| BMP-5 | 16-18^{Ob} | | 8.3^{T} | | YES | |
| BMP-6 | 16-18^{Ob} | 1? | 8.6^{T} | | YES | |
| BMP-7 | 16-18^{Ob} | 17.5 | 8.1^{T} | YES | YES | |
| | | | | | | |
| CDMP (pan) | | | | YES | YES | |

| | | | | | | |
|---|---|---|---|---|---|---|
| **Growth Factors** | | | | | | |
| | | | | | | |
| FGF-1 (acidic) | 16^{Ob} | 13 & 15 | 5.4 | | YES | |
| | | | | | | |

| **Bone Matrix Proteins** | | | | | | |
|---|---|---|---|---|---|---|
| | | | | | | |
| Osteocalcin | 5.8 | <10 | | YES | YES | |
| Osteonectin | 32 | 33 | | NO | YES | |
| BSP (BSP-11) | 35^{T} | | | | YES | |
| Lysyloxidase | | | | | | YES |
| Cathepsin L Pre | 37.4^{T} | 38 | 6.6 | | NO | YES |
| | | | | | | |

| **Serum Proteins** | | | | | | |
|---|---|---|---|---|---|---|
| | | | | | | |
| Albumin | 66^{T} | 68 | 5.6^{T} | YES | YES | |
| Serotransferrin Precursor | 76^{T} | 80 | 6.5^{T} | | YES | YES |
| Apo-A1-Lipoprotein | 28 | 36 | 5.6 | | YES | |
| Factor XIIIb Precursor | | 80 | | | | YES |

Additionally, intracellular proteins identified in Bone Protein include intracellular proteins: Dynein associated protein, protamine II, histone-like protein, L6 (ribosomal protein), and L32 (ribosomal protein). Other serum proteins that have been identified in BP include α2 microglobulin. Other extracellular matrix (bone matrix) proteins that have been identified include Frizzled related protein. All such proteins can be included, if desired, in a composition of the present invention.

Several preparations of protein mixtures or Bone Protein or derivatives thereof were examined to provide a gross estimate of the amount of several of the proteins in Table 6. Specifically, several different lots of BP and AX fractions that were extracted at pH 9.0, 9.5 and 10.0 (See Example 11) were examined by standard Western blot analysis using antibodies against TGFβ1, TGFβ2, BMP-3 and BMP-7. The resulting radiograph was scanned using a Sharp JX-330 scanner and the lane volume was calculated using ImageMaster ID software. To quantitate the amount of TGFβ1 in BP and its derivatives, recombinant bovine TGFβ1 was run as a standard (2, 4, 8 and 16 ng; Promega) and anti-bovine TGFβ1 was used. To calculate the amount of TGFβ1, TGFβ2, BMP-3, or BMP-7 in any given sample, the following formula was utilized: (lane volume sample/lane volume standard TGFβ1) X (quantity of standard TGFβ1 loaded). Anti-bovine TGFβ antibody was used. This method is a specific estimator of the amount of TGFβ1 in the samples and a gross estimator of the quantities of TGFβ2, BMP-3 and BMP-7 in the mixtures. Only gross estimates of TGFβ2, BMP-3 and BMP-7 can be made, because bovine standards and bovine antibodies for each of these three proteins are not available (human antibodies were used for these proteins, and quantities were estimated based on the TGFβ1 standard). The low and high quantity estimates for each of the four proteins over all of the compositions tested is shown in Table 6. For these compositions, the ratio (w/w) of TGFβ1 to all other proteins in the mixture is from about 1:1000 to about 1:100.

**TABLE 6**

| | TGFβ-1 | TGFβ-2 | BMP-3 | BMP-7 |
|---|---|---|---|---|
| Low quantity estimate (ng/µg protein in composition) | 0.9 | 8.4 | 3.8 | 1.5 |
| High quantity estimate (ng/µg protein in composition) | 9.8 | 114.0 | 89.2 | 43.5 |
| Low quantity estimate (% of total proteins) | 0.09 | 0.84 | 0.38 | 0.15 |
| High quantity estimate (% of total proteins) | 0.98 | 11.4 | 8.9 | 4.3 |

In another experiment, several preparations (6 lots) of Bone Protein (BP) were examined to provide a gross estimate of the amount of several of the proteins in Table 5. Specifically, several different lots of BP were examined by standard Western blot analysis using antibodies against TGFβ1, TGFβ2 and BMP-2. The resulting radiograph was scanned using a Sharp JX-330 scanner and the band volumes were quantified using ImageMaster ID software (Pharmacia Biotech), by selecting only the specific bands on the gel scan. The protein quantity was calculated using a linear curve equation from the standard curves. To quantify the amount of TGFβ1 in BP, recombinant human TGFβ1 was run as a standard (1, 2, 4 and 8 ng; Promega). To detect TGFβ1, anti-human TGFβ1 and an ALP-conjugated antibody were utilized as the primary and secondary antibodies, respectively. To quantify the amount of TGFβ2 in BP, recombinant human TGFβ2 was run as a standard (2, 4, 8 and 16 ng; Promega) and anti-human TGFβ2 was used. To quantify the amount of BMP-2 in BP, recombinant human BMP-2 was run as a standard (2, 4, 8 and 16 ng; Promega). To detect TGFβ2, anti-human TGFβ2 and an ALP-conjugated antibody were utilized as the primary and secondary antibodies, respectively. To quantify the amount of BMP-2, recombinant human BMP-2 was run as a standard (1.5, 3, 6 and 12 ng; NIBSC, WHP, Pottersbar, England). To detect BMP-2, anti-human BMP-2 and an ALP-conjugated antibody were utilized as the primary and secondary antibodies, respectively.

This method is a specific estimator of the amount of TGFβ1 and TGFβ2 in the samples and a gross estimator of the quantities of BMP-2 in the mixtures. Only gross estimates of BMP-2 can be made because bovine standards and bovine antibodies are not available, and the human and bovine BMP-2 sequences may differ. Those skilled in the art recognize that bovine and human TGFβ-1 have identical amino acid sequences and that and bovine and human TGFβ2 have identical amino acid sequences and, therefore, bovine TGFβ-1 and rhTGFβ-1 should have identical activities, as should bovine TGFβ2 and rhTGFβ2. Those skilled in the art also recognize that high purity TGFβ-1 can be isolated from bovine bone using methods disclosed by Seyedin (Ogawa et al., Meth. Enzymol., 198:317-327 (1991); Seyedin et al., PNAS, 82:2267-71 (1985)).

Results are shown in Table 7.

**TABLE 7**

| | TGFβ-1 | TGFβ-2 | BMP-2 |
|---|---|---|---|
| Low quantity estimate (ng/µg protein in composition) | 0.7 | 13 | 3.2 |
| High quantity estimate (ng/µg protein in composition) | 1.35 | 35 | 16.1 |
| Low quantity estimate (% of total proteins) | 0.07 | 1.3 | 0.32 |
| High quantity estimate (% of total proteins) | 0.14 | 3.5 | 1.61 |

### Example 6

The following example demonstrates the bone- and cartilage-inductive activity of bone protein (BP) and protein mixtures derived from the complex mixture of proteins in BP as compared to individual recombinant protein components, as determined using a standard *in vivo* rodent subcutaneous assay.
A. In the following experiment, a rodent subcutaneous assay and grading system developed by Sulzer Biologics, Inc. (Denver, Colorado), was used to evaluate the cartilage-and bone-inductive capabilities of recombinant BMP-2 (provided to Sulzer Biologics, Inc. by Genetics Institute). The results were then compared to data for BP which was generated in the same assay and using the same grading system.
To perform the Sulzer Biologics, Inc. rat subcutaneous assay, a matrix is prepared from a suitable material. When the material was type I collagen, a dispersion of 4% w/w collagen was prepared using bovine type I collagen and 1% glacial acetic acid. Collagen discs/sponges were formed in molds, and then frozen and lyophilized. For the assay, the discs were loaded with the composition (e.g., bone protein, a subset of bone protein, a growth factor) by incubation of the disc with the composition at room temperature for about 30 minutes, followed by freezing and lyophilization of the discs. The discs were implanted into Long-Evans rats in subcutaneous positions. In all of the experiments described in this example and other examples below, 5-10 rats were used per treatment and the composition treated collagen disc/sponge (or other material in Example 14) was implanted for three weeks. At the end of three weeks, the rats were euthanized, and the explants were surgically removed, histologicallyprocessed, and graded. The grading scale utilized for bone-inductive activity in the rodent subcutaneous assay, which was developed by Sulzer Biologics, Inc., is shown in Table 1 (See Example 1). The grading scale utilized for cartilage-inductive activity in the rodent subcutaneous assay, which was also developed by Sulzer Biologics, Inc., is shown in Table 8.

**TABLE 8**

| A | B | C | D | E |
|---|---|---|---|---|
| 0 | No | N/A | N/A | N/A |
| 1 | Yes | No | No | No |
| 2 | Yes | Yes | No | No |
| 3 | Yes | Yes | Yes | No |
| 4 | Yes | Yes | Yes | Yes |

| | | | | |
|---|---|---|---|---|
| KEY: A=Score; B=Presence of mineralized tissue; C=some non-mineralized cartilage; D=non-mineralized cartilage area is comparable to bone area; cartilage is in ring >50% of circumference of explant; often heavy cartilage stain; E=non-mineralized cartilage area is greater than bone area; often very little or no mineralization resulting from bone or cartilage. | | | | |

BP, administered at a dose of 10 µg on a collagen sponge, routinely scores between 1.5 and 2.2 for cartilage, and between 2.0 and 2.5 for bone. More specifically, in the rat subcutaneous assay, BP induces an ordered ring of bone formation around the periphery of the collagen sponge. BP and BP derivatives described in the examples below (See Examples 7 and 8) produce an ordered ring of bone on the outside and just interior, an ordered cartilage ring. Some derivatives are more efficient at producing this inner cartilage ring than others. AX fractions that were eluted at pH 9.0, 9.5 and 10.0, and BP are efficient at inducing this inner cartilage ring. Additionally, BP with exogenously added TGFβ, such as TGFβ1-3 and preferably, TGFβ1, wherein the ratio of TGFβ1 to BP is as high as 1:10 (e.g., 1 µg exogenously added TGFβ1 to 10 µg BP), also is efficient at producing this ordered ring of bone on the outside and just interior, an ordered cartilage ring. Without being bound by theory, the present inventors believe that this characteristic provides a superior tendon to bone fixation.
In contrast, recombinant human BMP-2 gave the results shown in Table 9, revealing that BMP-2 has both a lower bone and cartilage score as compared to BP. It is noted that BMP-2 does not induce the ordered ring of bone formation around the periphery of the collagen sponge, nor, just interior, an ordered cartilage ring.

**TABLE 9**

| BMP-2 Rat subcutaneous assay | | | |
|---|---|---|---|
| | 1.0 µg | 3.5 µg | 10 µg |
| Bone Score | 1.2 ± 0.1 | 1.3 ± 0.1 | 1.7 ± 0.2 |
| Cartilage Score | 1.0 ± 0.0 | 1.0 ± 0.0 | 1.0 ± 0.0 |

B. TGFβ-1 and -2 were initially identified by the ability to stimulate chondrogenesis *in vitro.* However, it is known in the art that TGFβ1-5, and growth factors such as FGF and PDGF, do not induce bone or cartilage in the rat subcutaneous assay, such as the *in vivo* rodent ectopic assay (e.g., both TGFβ-1 and -2 are unable to initiate cartilage or bone formation; only fibrous tissue is observed). This is confirmed by the results shown below with recombinant TGFβ1. The assay was performed using Sulzer Biologics, Inc. rat subcutaneous assay and grading system as described in section (A) above. Table 10 shows that recombinant human TGFβ1 does not induce bone or cartilage in this assay. In contrast, Bone Protein (BP), administered at a dose of 10 µg, routinely scores between 1.5 and 2.2 for cartilage, and between 2.0 and 2.5 for bone (see examples below).

**TABLE 10**

| TGFβ1 Rat subcutaneous assay | | |
|---|---|---|
| | 10 µg | 35 µg |
| Bone Score | 0.0 ± 0.0 | 0.0 ± 0.0 |
| Cartilage Score | 0.0 ± 0.0 | 0.0 ± 0.0 |

### Example 7

The following example demonstrates that mixtures of proteins derived from a modified purification process for BP that contains BMP-2, BMP-3, BMP-7, and TGFβ-1 produce both bone and cartilage.

BP is normally purified using anion exchange (AX), cation exchange (CX) and high performance liquid chromatography (HPLC) steps (PCT Publication No. W095/13767, incorporated herein by reference in its entirety). Such a method is described in detail above and in U.S. Patent No. 5,290,763, incorporated herein by reference in its entirety.

Typically, proteins are eluted from the AX column at pH=8.5. For this experiment, proteins were eluted from the AX column at pH 9.0, 9.5, and 10.0. The samples were then purified across the CX and HPLC columns as described previously (PCT Publication No. W095/13767). Each HPLC fraction that was eluted at the above pHs was assayed by Western blot for the presence of BMP-2, BMP-3, BMP-7, TGFβ-1, and TGFβ-2.

Elution of the AX column with increasing pH resulted in increased quantity of BMP-2, BMP-3, BMP-7, and TGFβ-1 in BP. At a pH of 9.0, BMP-2 and BMP-7 quantities show the greatest increase, whereas TGFβ-1 and BMP-3 show limited or no increase compared to pH 8.5. Increasing quantities of these factors have no significant effect on the bone score and slightly increase the cartilage score (Table 12). At pH 10, the TGFβ-1 quantity increases much more than the increase for BMP-2, -3, and -7, and the pH 10 fraction shows the greatest cartilage score. The HPLC purified fractions (10 µg) were tested in the rat subcutaneous model, the results of which are shown in Table 11.

**TABLE 11**

| pH | 8.5 | 9.0 | 9.5 | 10.0 |
|---|---|---|---|---|
| Bone Score | 2.0±0.0 | 2.2±0.2 | 2.0±0.0 | 2.1±0.3 |
| Cartilage Score | 1.5±0.2 | 2.0±0.1 | 1.9±0.1 | 2.3±0.1 |

### Example 8

The following example demonstrates that a composition purified from BP and including BMP-2, BMP-3, BMP-7 and TGFβ-1, contains components required for bone and cartilage formation.

To obtain specific protein subsets of BP, proteins within BP were separated on a hydroxyapatite column. The void material contained proteins that eluted with <120 mM KPO₄ buffer. During the elution, the pH gradient ranged from 6.0-7.4. 10 µg of each fraction was added to collagen sponges and the rodent subcutaneous assay was performed as described in Example 6 above. In addition, an equivalent quantity of each fraction was loaded onto a protein gel and a Western blot was performed with antibodies against BMP-2, BMP-3, BMP-7, and TGFβ-1 (Table 12). A (-) indicates no signal detected and a (+) indicates that a signal was detected.

**TABLE 12**

| Marker | Void | Peak | Wash | BP |
|---|---|---|---|---|
| BMP-2 | - | + | + | + |
| BMP-3 | - | + | + | + |
| BMP-7 | - | + | + | + |
| TGFβ-1 | - | + | + | + |

The data in Table 14 demonstrates that a BP fraction containing BMP-2, 3, 7 and TGFβ-1, contains proteins which are required components for BP cartilage and bone inductive activity.

**TABLE 14**

| Measure | Void | Peak | Wash | BP |
|---|---|---|---|---|
| Bone score | 0.2 | 2.4 | 2.4 | 2.2 - 2.5 |
| Cartilage score | 0.0 ± 0.0 | 2.4 ± 0.2 | 2.0 ± 0.0 | 2.2 ± 0.2 |

### Example 9

The following example demonstrates that different cartilage repair matrices combined with BP induce bone and cartilage formation *in vivo* according to the present invention.
A. This example demonstrates the use of a poly lactic acid:polyglycolic acid material that contains BP for bone and cartilage formation.
A 60% (v/v) solution of polylactic acid: polyglycolic acid (PLGA) (50:50) in N-Methyl Pyrrilidone was made. Collagen (6 mg) was pressed in a Delrin mold. The PLGA (140 mg) was added and mixed. BP (100 µg) contained in a 10 mM HCl solution (100 microliters) was added and all components were mixed together to form a solid disc. This mixture was pressed into a mold and incubated at room temperature for one hour and then implanted into rats in the rat subcutaneous assay as described in section 5. Histology was performed after one month of implantation. Table 15 shows that BP combined with the PLGA matrix induces bone and cartilage formation in this *in vivo* model.

**TABLE 15**

| | PLGA-collagen-BP |
|---|---|
| Bone score | 2.6 ± 0.7 |
| Cartilage Score | 1.2 ± 0.5 |

B. In this assay, a 3% collagen Type I/ 1% collagen Type IV (Sigma) composite sponge was made using the normal preparation method as described in Example 4 of the application. This matrix, containing 10 µg BP, was implanted into rats in the rat subcutaneous assay as described in section 5, and histology was performed after three weeks. Table 16 shows that BP combined with the matrix containing both type I and type IV collagen induces bone and cartilage formation in this *in vivo* model.

**TABLE 16**

| | Type I/IV |
|---|---|
| Bone Score | 1.2 ± 0.2 |
| Cartilage Score | 1.2 ± 0.2 |

C. This experiment demonstrates that cartilage repair matrices containing triethanolamine and collagen in the form of a gel (basic pH), combined with BP, induce cartilage formation *in vivo.*
For basic collagen preparation, 0.5 gram TEA was added to 99.5 gram dI water to pH 9.9. To make a 2% collagen gel, 2.0 gram collagen was added to pH 8.8. The mixture was incubated at room temperature for one hour and then frozen and lyophilized 48 hours. The mixture was reconstituted in 10 mM HCL (containing 35 micrograms of BP) as 3 and 6% collagen sponges. The gel was delivered through a #18 or #20 needle in a 100 microliter volume. After drying, the resulting sponges were 80% collagen and 20% TEA by weight. Table 17 demonstrates that both 3 and 6% collagen TEA sponges combined with BP induce bone and cartilage in this *in vivo* system.

**TABLE 17**

| AED | 6% collagen TEA + 35 µg BP | 3% collagen TEA + 35 µg BP |
|---|---|---|
| Bone Score | 3.0 ± 0.8 | 2.3 ± 1.0 |
| Cartilage Score | 2.3 ± 0.3 | 2.3 ± 0.3 |

D. This experiment demonstrates that cartilage repair matrices containing collagen at acidic pH in the form of an injectable gel, combined with BP, induce cartilage formation *in vivo.* A BP solution (1 mg/ml) contained in 10 mM HCl (5 ml) was added to collagen (30 mg/ml). The solutions were mixed until a jelly-like consistency and were incubated at 4°C overnight. In the absence of an incision, 100 microliters of the gel was injected subcutaneously into the rat. Table 18 demonstrates that BP in a matrix in the form of a gel induces bone and cartilage formation in this *in vivo* model.

| | Collagen gel + 100 µg BP |
|---|---|
| Bone score | 3.5 ± 0.5 |
| Cartilage Score | 2.0 ± 1.0 |

E. This experiment demonstrates that cartilage repair matrices containing collagen at acidic pH in the form of an injectable gel, combined with BP, induce cartilage formation *in vivo.* This experiment differs from part D above in that 1 M H₃PO₄, instead of HCl, was used. To prepare the acidic pH collagen gel, to 995 ml dI water, 5.0 ml 1 M H₃PO₄ was added, followed by 1.75 ml 1.00 N NaOH (mixture pH = 2.49). 10.0 g collagen was added to BP and mixed for 2 hours (final pH = 3.61). This makes a 1% collagen gel, which was frozen and lyophilized. A 60 mg collagen sponge was loaded with 2 ml dI water and incubated 2 hours at room temperature. The resulting 3% collagen gel had a pH of 3.7. The material was injectable using #18 and #20 gauge needles, but not with #22 or #25 gauge needles. A 100 microliter volume contained 35 µg BP. Table 19 demonstrates that 3 % acidic collagen matrices in the form of a gel and combined with BP induce bone and cartilage in this *in vivo* system.

**TABLE 19**

| AED | 3% collagen + 35 µg BP |
|---|---|
| Bone score | 3.0 ± 0.0 |
| Cartilage score | 1.8 ± 0.3 |

While various embodiments of the present invention have been described in detail, it is apparent that modifications and adaptations of those embodiments will occur to those skilled in the art. It is to be expressly understood, however, that such modifications and adaptations are within the scope of the present invention, as set forth in the following claims:

## Claims

1. The use of a mixture of proteins for the manufacture of a medicament for enhancing an attachment of bone to connective tissue, the mixture comprising at least four different members of the TGFβ superfamily proteins.

2. Use according to claim 1, wherein the mixture comprises at least one of TGFβ1, TGFβ2, TGFβ3, TGFβ4 and TGFβ5.

3. Use according to any preceding claim, wherein the mixture comprises at least one of BMP-2, BMP-3, BMP-4, BMP-5, BMP-6, BMP-7 and BMP-8.

4. Use according to any preceding claim, wherein the mixture comprises at least one bone matrix protein.

5. Use according to claim 4, wherein the at least one bone matrix protein is selected from osteocalcin, osteonectin, bone sialoprotein (BSP), lysyloxidase, cathepsin L pre, osteopontin, matrix GLA protein (MGP), biglycan, decorin, proteoglycan-chondroitin sulfate III (PG-CS III), bone acidic glycoprotein (BAG-75), thrombospondin (TSP) and fibronectin.

6. Use according to any preceding claim, wherein the mixture comprises at least one growth factor protein.

7. Use according to claim 6, wherein the at least one growth factor protein is selected from fibroblast growth factor I (FGF-I), FGF-II, FGF-9, leukocyte inhibitory factor (LIF), insulin, insulin growth factor I (IGF-I), IGF-II, platelet-derived growth factor AA (PDGF-AA), PDGF-BB, PDGF-AB, stromal derived factor-2 (SDF-2), pituitary thyroid hormone (PTH), growth hormone, hepatocyte growth factor (HGF), epithelial growth factor (EGF), transforming growth factor-α (TGFα) and hedgehog proteins.

8. Use according to any preceding claim, wherein the mixture comprises at least one serum protein.

9. Use according to claim 8, wherein the at least one serum protein is selected from albumin, transferrin, α2-Hs GlycoP, IgG, α1-antitrypsin, β2-microglobulin, Apo A1 lipoprotein (LP) and Factor XIIIb.

10. Use according to any preceding claim, wherein the mixture comprises at least one bone derived protein.

11. Use according to claim 10, wherein the at least one bone derived protein is derived from human bone.

12. Use according to any preceding claim, wherein the mixture comprises at least one exogenous protein.

13. Use according to any preceding claim, wherein the mixture comprises TGFβ1, BMP-2, BMP-3 and BMP-7.

14. Use according to any preceding claim, wherein the medicament additionally comprises a matrix configured to interface between connective tissue and bone.

15. Use according to claim 14, wherein the matrix is bioresorbable.

16. Use according to claim 14 or 15, wherein the matrix is porous.

17. Use according to any one of claims 14 to 16, wherein the matrix comprises a material selected from the group consisting of a synthetic polymeric material and a ground substance.

18. Use according to any one of claims 14 to 17, wherein the matrix comprises a material selected from the group consisting of a sponge, a membrane, a film and a gel.

19. Use according to any one of claims 14 to 18, wherein the matrix comprises collagen from bovine tendon.
